# EUROPEAN PATENT APPLICATION

(11) **EP 3 549 927 A1**
(43) Date of publication of application: **09.10.2019**
(21) Application number: 18305407.1
(22) Date of filing: 06.04.2018
(51) Int. Cl.: C07B 59/00

(54) **A PROCESS FOR THE SYNTHESIS OF CARBON LABELED ORGANIC COMPOUNDS**

(71) Applicant: Commissariat à l'Energie Atomique et aux Energies Alternatives, 75015 Paris (FR)
(72) Inventor: AUDISIO, Davide, 91300 MASSY (FR); CANTAT, Thibault, 92130 ISSY LES MOULINEAUX (FR); DESTRO, Gianluca, 91400 ORSAY (FR)
(74) Representative: Gevers & Orès

(57) **Abstract**

The present invention relates to a process for the synthesis of a carbon labeled organic compound containing a carbon labeled carboxyl group.

The present invention also concerns the use of carbon labeled organic compounds containing a carbon labeled carboxyl group obtained by the process of the invention, in the manufacture of pharmaceuticals and agrochemicals, in particular pharmaceuticals and agrochemicals having a free carboxylic acid functionality.

Another aspect of the invention relates to a process for manufacturing labeled pharmaceuticals and agrochemicals, in particular pharmaceuticals and agrochemicals having a free carboxylic acid functionality, comprising a step of synthesis of carbon labeled organic compounds containing a carbon labeled carboxyl obtained by the process of the invention

A still another aspect of the invention further relates to a process for producing tracers and radiotracers, characterized in that it comprises a step of synthesis of carbon labeled organic compounds containing a carbon labeled carboxyl group obtained by the process of the invention.

## Description

The present invention relates to a process for the synthesis of a carbon labeled organic compound containing a carbon labeled carboxyl group.

The present invention also concerns the use of carbon labeled organic compounds containing a carbon labeled carboxyl group obtained by the process of the invention, in the manufacture of pharmaceuticals and agrochemicals, in particular pharmaceuticals and agrochemicals having a free carboxylic acid functionality.

Another aspect of the invention relates to a process for manufacturing labeled pharmaceuticals and agrochemicals, in particular pharmaceuticals having a free carboxylic acid functionality, comprising a step of synthesis of carbon labeled organic compounds containing a carbon labeled carboxyl obtained by the process of the invention

A still another aspect of the invention further relates to a process for producing tracers and radiotracers, characterized in that it comprises a step of synthesis of carbon labeled organic compounds containing a carbon labeled carboxyl group obtained by the process of the invention.

Carbon is a chemical element with four main isotopes: carbon-11 (¹¹C), carbon-12 (¹²C), carbon-13 (¹³C) and carbon-14 (¹⁴C). Three isotopes are naturally occurring isotopes of carbon: ¹²C, ¹³C and ¹⁴C. ¹²C and ¹³C are stable, occurring in a natural proportion of approximately 99:1. ¹⁴C constitutes a negligible part, but since it is radioactive with a half-life of 5,700 years, it is radiometrically detectable.

Except for carbon-12 (¹²C), all the other isotopes have potentials in major applications. The labeling of an organic molecule with carbon-11 (¹¹C), carbon-13 (¹³C) and carbon-14 (¹⁴C) represents a major synthetic challenge.

**Carbon-13** is a natural, stable isotope of carbon widely used in NMR spectroscopy. Carbon-13 (¹³C) labeled molecules are utilized as internal standards for mass spectroscopy studies.

In recent years, ¹³C magnetic resonance spectroscopy (MRS), which can detect signals from multiple cellular metabolites following administration of a ¹³C-labeled substrate, has been widely used to follow metabolic processes *in vivo.* The recent development of dynamic nuclear polarization (DNP), which dramatically increases the sensitivity of the ¹³C MRS experiment (>10,000 times) has allowed real-time imaging of several substrates and the metabolites formed therefrom *in vivo.*

**Carbon-14** is a radioactive isotope of carbon with an atomic nucleus containing 6 protons and 8 neutrons. ¹⁴C is a long-lived isotope (half-life 5730 years). Its presence in organic materials is the basis of the radiocarbon dating method to date archaeological, geological and hydrogeological samples. Compounds labeled with carbon-14 are frequently used as tracers during the development of potential new drug candidates to aid in the understanding of their absorption, distribution, metabolism, and excretion properties. While compounds labeled with tritium are used during basic research studies, compounds labeled with carbon-14 are preferred for definitive drug metabolism and pharmacokinetics studies because their positions are inherently more resistant to cleavage from metabolic biotransformation. However, they are much more expensive to synthetize, requiring many more synthetic steps that result in the increased expenditure of time and effort.

**Carbon-11,** a nearly pure positron emitter (t_{1/2} = 20.38 min, E_{avg}(*β*⁺) = 0.39 MeV) is used extensively for developing radiotracers for positron emission tomography (PET), a non-invasive molecular imaging technique. The development of PET radiopharmaceuticals may provide an ideal methodology to enable diagnosis, monitor disease progression, and evaluate drug therapies in vivo without eliciting a pharmacological response. In addition to serving as a clinical tool for disease diagnosis, PET is increasingly relevant for drug development as it can provide quantitative pharmacokinetic, biodistribution, and receptor occupancy data for a drug candidate.

The labeling of organic molecules with carbon isotopes ¹³C, ¹⁴C and ¹¹C represents today a challenge and limits the number of molecules that can be labeled.

From a synthetic chemical standpoint, the insertion into an organic molecule of a carbon isotope (¹¹C, ¹³C, and ¹⁴C) is a challenging procedure and often a major limitation for the utilization of carbon labeled compounds in all possible applications.

The main reason is that the basic building block available for labeling is carbon dioxide (CO₂): a simple gas molecule, which is thermodynamically and kinetically very stable, and demands remarkable drastic conditions for its functionalization. To date, the approaches described in the literature for the derivatization of CO₂ require long multi-step and time demanding procedures, which are not compatible with the demands of carbon isotope chemistry.

For carbon-14 (¹⁴C), multi-step synthesis is effective but unfortunately it generates large amounts of radioactive waste (T_{1/2} = 5700 years), which must be treated separately and are extremely expensive to deal with. This is the reason why the synthesis with carbon-14 is very expensive, time consuming and represents a major problem for pharmaceutical and agrochemical industries. If the economics of time and cost were to be significantly lowered, use of carbon-14 tracers would prevail by virtue of the higher quality information they afford leading to better candidate selection/deselection at much earlier stages of drug development, before greater investments are made by the pharmaceutical industry.

For carbon-11 (¹¹C), the major challenge is represented by its very short half-life (T_{1/2 C)}= 20 min). Only a few effective methods exist today to label molecules with this isotope. These methods are mostly limited to the methylation of heteroatoms. Carbon-11 is most often incorporated into small molecules by methylation of alcohol, thiol, amine or carboxylic acid precursors using [¹¹C]methyl iodide or [¹¹C]methyl triflate (generated from [¹¹C]carbon dioxide). Consequently, small molecules that lack an easily substituted ¹¹C-methyl group are often considered to have non-obvious strategies for radiolabeling and require a more customized approach. PET radiochemists are very often forced to do pharmacomodulations of the drug and to label it with other isotopes (¹⁸F).

For effective tracking of a drug molecule throughout a complete physiological system, the use of radiolabeled drugs enables both a qualitative and quantitative assessment of drug distribution, metabolism, and excretion (ADME). Either ¹⁴C (carbon-14) or ³H (tritium) are used as radioactive isotopes in such ADME studies, with a preference usually for ³H for early *in vitro* assays driven by the cheaper preparation of such species, and ¹⁴C for later *in vivo* studies, in virtue of its biological stability, in addition to the potential risk of losing a tritium label upon oxidative biotransformation and the possibility of inducing metabolic isotope effects.

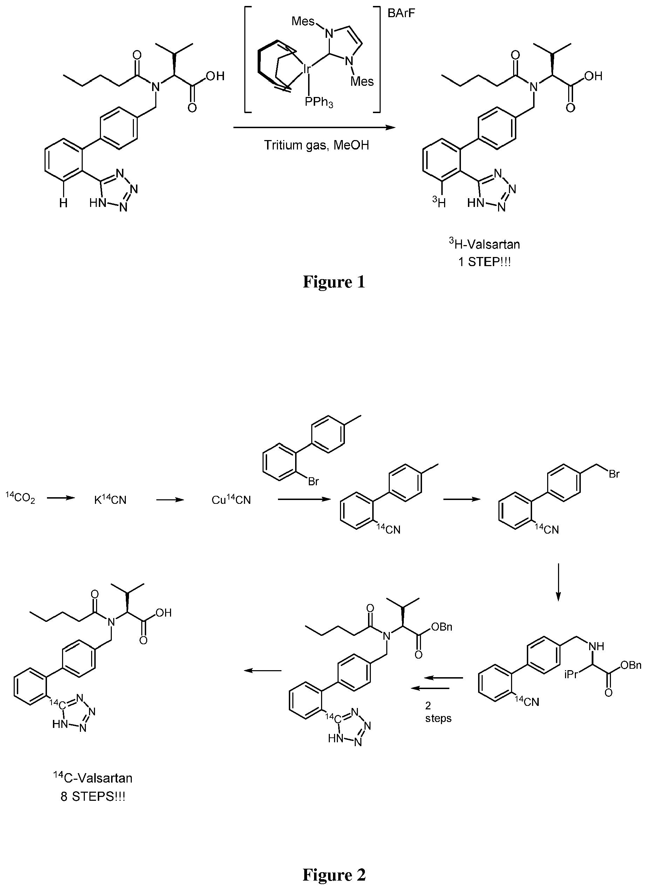

In recent years, the development of new C-H activation reactions catalyzed by transition metals had a huge impact on tritiation of organic molecules. There are now a large variety of methods that allow to selectively perform Hydrogen (¹H)/Tritium (³H) exchange in one single operation, using tritium gas (T₂), a readily available source of tritium. The labeling of valsartan (angiotensin II receptor antagonist) is a representative example. It is possible to label valsartan with ³H in one single operation using the appropriate catalyst and readily available valsartan as shown in Figure 1.

The synthesis of carbon-14 labeled compound is usually performed through lengthy multi-step processes from carbon dioxide (CO₂), the basic and simplest building block available for labeling. Unfortunately, these processes are costly and generate huge amounts of radioactive wastes. Carbon dioxide is converted systematically into a more and more complex building block until its conversion to the desired molecule.

A representative example is the synthesis of carbon-14 labeled valsartan (an angiotensin II receptor antagonist) reported by Novartis Pharma in 2000 (Moenious *et al*.) and illustrated in Figure 2. As shown in Figure 2, the synthesis requires several synthetic steps from carbon dioxide. It is worth noting that cyanide anion is prepared from CO₂ (as BaCO₃) using a very hazardous method as reported by: Voges, R.; Heys, J. R.; Moenius, T. in "Preparation of Compounds Labeled with Tritium and Carbon-14", John Wiley & Sons, Ltd, 2009, 393:
"*An intimate mixture of barium [¹⁴C]-carbonate, potassium azide and carefully dried sea sand is heated at temperatures slowly increasing from 450 to 700 °C. The resulting crude product is acidified with 85% phosphoric acid, and H¹⁴CN released is expelled with helium into a methanolic solution of potassium methoxide, from which the K¹⁴CN is isolated in solid form by evaporation of the solvent. (Caution: HCN is volatile and extremely toxic.)"*

The synthesis of ¹⁴C labeled valsartan is thus much longer, time consuming and hazardous compared to the synthesis of ³H labeled valsartan illustrated in Figure 1.

Among the advantages in having ¹⁴C labeled compounds rather than ³H labeled compounds, are
- the higher metabolic stability of the ¹⁴C labeled compounds: the tritium C-³H bonds can be oxidized and metabolized more easily than the C-¹⁴C bonds;
- no isotopic effect can be expected: tritium is three times the size of hydrogen (Chem. Res. Toxicol. 2012, 25, pp. 513-531; J. Label. Compd. Radiopharm 2015, 56, p. 441).

Despite their high interest, ¹⁴C labeled compounds are much more expensive to synthesize, requiring several synthetic steps that result in the increased expenditure of time and effort.

Consequently, the development a direct insertion of ¹⁴C would be highly beneficial and rationalize the synthesis of carbon-14 labeled compounds. As mentioned, in the literature only sequential multi-step carbon isotope insertions are known and no direct insertion of ¹⁴C is reported.

There is a thus need to develop a process for the synthesis of carbon isotopically labeled compounds that addresses the drawbacks of the art.

In particular, thus need to develop a process for the synthesis of carbon isotopically labeled compounds, more specifically a carbon labeled organic compound containing a carbon labeled carboxyl group, that allows a direct insertion of carbon isotopes using a readily available source of carbon-11(¹¹C), carbon-13(¹³C), or carbon-14(¹⁴C).

More particularly, there is a need for a process for the synthesis of a carbon labeled organic compound containing a carbon labeled carboxyl group that, unlike the multi-step processes of the art, involves a significantly reduced number of steps, ideally only one step, using a readily available source of carbon-11(¹¹C), carbon-13(¹³C), and carbon-14(¹⁴C).

Additionally, there is a need for a process for the synthesis of a carbon labeled organic compound containing a carbon labeled carboxyl group as described above, that uses reactants that are relatively non-toxic, easy to handle, commercially available and/or can easily be synthesized, and which can be carried out under mild conditions, in particular, under conditions that tolerate the presence of structural groups borne by the compounds and reactants involved in the process of the invention.

The present invention addresses these needs among others by providing a process for the synthesis of a carbon labeled organic compound containing a carbon labeled carboxyl group according to formula (I) 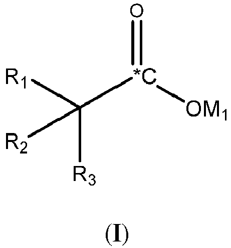 wherein
▪ *C is a ¹¹C, ¹³C or ¹⁴C isotope;
▪ R₁, R₂ and R₃ are, independently, a hydrogen atom, an aryl, a heteroaryl, a heterocycle, an alkyl, an alkyl halide, an alkene or an alkyne, said aryl, heteroaryl, heterocycle, alkene, alkyne and alkyl groups being optionally substituted, or
   R₁ and R₂ form together with the carbon atom to which they are linked a carbonyl (-(C=O)- and R₃ is as defined above, or
   R₁ and R₂ form together with the carbon atom to which they are linked an alkene having at least one double bond, with one double bond being alpha to the carboxyl group, said alkene being optionally substituted, and R₃ is as defined above, or
   R₁, R₂ and R₃ form together with the carbon atom to which they are linked an aryl, a heteroaryl, or a heterocycle, said aryl, heteroaryl and heterocycle being optionally substituted;
▪ M₁ is a hydrogen atom, a silver cation (Ag⁺), an alkaline cation selected from lithium (Li⁺), sodium (Na⁺), potassium (K⁺), rubidium (Rb⁺), or cesium (Cs⁺);
characterized in that
- an organic compound containing a carboxyl group according to formula (II) 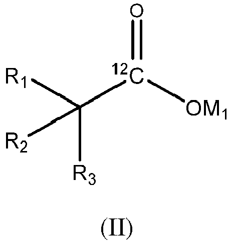 wherein R₁, R₂, R₃ and M₁ are as defined above,
- is reacted with a labeled *CO₂ wherein *C is an isotope as defined above,
- in the presence of a catalyst system comprising an inorganic salt of formula (III)

   M₂(L)ₘ (III)

   wherein
   - M₂ is a transition metal selected from Cu, Pd, Ni, Ru, Ag, Rh, Fe, Co, Zn, Ir, Au, Pt,
   - m is 1 or 2;
   - L is a halogen atom selected from fluorine, chlorine, bromine, and iodide, a triflate or trifluoromethylsulfonate, a tosylate or p-toluenesulfonate, a mesylate or methanesulfonate, -CN,
   and a ligand of formula (IV), formula (V) or formula (VI) 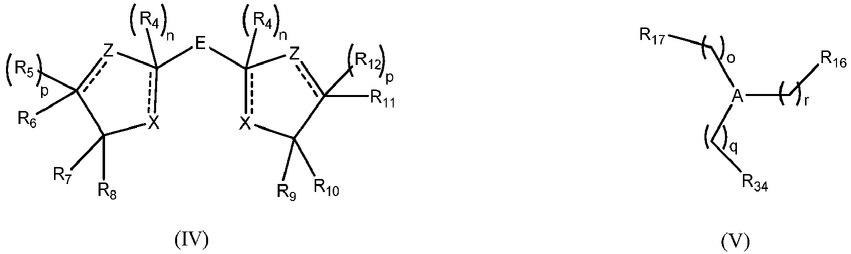 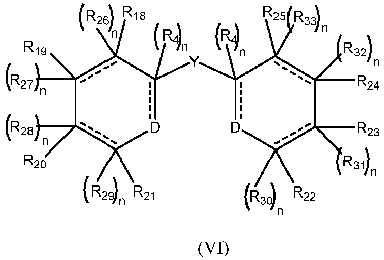 wherein
   - n is 0 or 1;
   - n' is 0 or 1;
   - n" is 0 or 1;
   - n'" is 0 or 1;
   - p is 0 or 1;
   - o is 0 or 1;
   - q is 0 or 1;
   - r is 0 or 1;
   - t is 0, 1, 2 or 3;
   - E is a single bond, 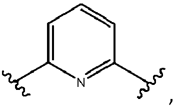 -C(R₁₃R₁₄)- with R₁₃ and R₁₄, independently being a hydrogen atom, an alkyl, an aryl, -CN, -NO₂, a halogen atom selected from F, Cl, Br, I;
   - X is N(R₁₅)_{n'}, O, P, S(R₁₅)_{n'}, or P(R₁₅)_{n'} with R₁₅ being a hydrogen atom or an alkyl and with the proviso that when X is N(R₁₅)_{n'}, S(R₁₅)_{n'}, or P(R₁₅)_{n'}, and n'=n=0, ---- is a double bond;
   - Z is N(R₁₅)_{n"}, O, P, S(R₁₅)_{n"}, or P(R₁₅)_{n"}, with R₁₅ being a hydrogen atom or an alkyl and with the proviso that when Z is N(R₁₅)_{n"}, S(R₁₅)_{n"}, or P(R₁₅)_{n"}, and n"=p=0, ---- is a double bond;
   - A is N or P;
   - Y is a single bond, 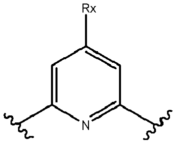 with Rₓ being a hydrogen atom or an alkyl;
   - D is N(R₁₅)_{n'"}, O, P, S(R₁₅)_{n"'}, or P(R₁₅)_{n"'} with R₁₅ being a hydrogen atom or an alkyl and with the proviso that when D is N(R₁₅)_{n'"}, S(R₁₅)_{n'"}, or P(R₁₅)_{n'"} and n'"=n=0, ---- is a double bond;
   - R₄, R₅, R₆, R₇, R₈, R₉, R₁₀, R₁₁ and R₁₂ are, independently, a hydrogen atom, an alkyl, an alkoxy, an aryl, or -CN, said alkyl and aryl being optionally substituted, or
      R₄, R₅, R₈, R₉, and R₁₂ are, independently, a hydrogen atom, an alkyl, an alkoxy, an aryl, or -CN, R₆ and R₇ and/or R₁₀ and R₁₁ form together with the carbon atoms to which they are linked a heterocycle, said alkyl, aryl and heterocycle being optionally substituted;
   - R₁₈, R₁₉, R₂₀, R₂₁, R₂₂, R₂₃, R₂₄, R₂₅, R₂₆, R₂₇, R₂₈, R₂₉, R₃₀, R₃₁, R₃₂ and R₃₃ are, independently, a hydrogen atom, an alkyl, an alkoxy, an aryl, or a -CN, said alkyl and aryl being optionally substituted, or
      when Y is a single bond, n=0, ---- is a double bond, R₁₈, R₂₁, R₂₂, R₂₅, R₂₆, R₂₇, R₂₈, R₂₉, R₃₀, R₃₁, R₃₂ and R₃₃ are, independently, a hydrogen atom, an alkyl, an alkoxy, an aryl, or -CN, and R₁₉ and R₂₀ and/or R₂₃ and R₂₄ form together with the carbon atoms to which they are linked an aryl, said alkyl and aryl being optionally substituted;
   - R₁₆, R₁₇ and R₃₄ are, independently, a hydrogen atom, an alkyl, an alkoxy, an aryl, a heteroaryl, a heterocycle, said alkyl, aryl, heteroaryl and heterocycle being optionally substituted, -(C=S)-NR₃₅R₃₆, -(C=O)-NR₃₅R₃₆, -(CH₂)ₜ-NR₃₅P₃₆, -(CH₂)ₜ-PR₃₅R₃₆ with
      R₃₅ and R₃₆ being independently, a hydrogen atom, an alkyl, an alkene, an alkyne, an aryl, a heteroaryl, a heterocycle, said alkyl, alkene, alkyne, aryl, heteroaryl and heterocycle being optionally substituted,
      R₃₅ and R₃₆ form together with the nitrogen atom to which they are linked an optionally substituted heteroaryl or heterocycle.

The synthesis of a carbon labeled organic compound containing a carbon labeled carboxyl group of formula (I) by the process of the invention involves only one step.

The use of carbon labeled CO₂ as the source of carbon-11(¹¹C), carbon-13(¹³C), or carbon-14(¹⁴C) to directly label an organic compound containing a carboxyl group or a pharmaceutical drug containing a carboxyl group without modifying its structure, in only one synthetic step, is a great benefit in carbon radiochemistry as it:
- reduces radioactive wastes,
- reduces the reaction time of the synthesis,
- reduces the cost of the synthesis,
- gives access to new drugs which are not possible to label with the current known technologies.

As used herein, and unless otherwise indicated, the term "ligand" means a molecule that binds to a central metal atom to form a coordination complex. The bonding with the metal generally involves formal donation of one or more of the ligand's electron pairs. The nature of metal-ligand bonding can range from covalent to ionic. In general, ligands are viewed as electron donors and the metals as electron acceptors.

As used herein, and unless otherwise indicated, the term "alkyl" means a saturated, monovalent, unbranched, branched or cyclic hydrocarbon having C₁-C₂₄, for example, C₁-C₈ carbon atoms. Examples of alkyls include, but are not limited to, methyl, ethyl, propyl, butyl, pentyl, hexyl, octyl, nonyl, decyl, undecyl, dodecanyl and their branched isomers such as isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl. Examples of cyclic alkyls include, but are not limited to, cyclopropyl, cyclobutyl, cyclopentyl, cyclohexyl, bicylco[2,1,1] hexyl, bicyclo[2,2,1] heptyl, cyclopropylmethyl. An alkyl can be unsubstituted or substituted with one or more suitable substituents selected among halogen atoms such as fluorine, chlorine, bromine, iodine; hydroxyl; alkoxy; alkyl; alkylhalides; nitro (-NO₂); nitrile (-CN); and aryl, with alkyl as defined above and alkylhalides, alkoxy and aryl as defined hereinafter. A non-limiting example of an alkyl substituted by an aryl is a benzyl (-CH₂-C₆H₅).

As used herein, and unless otherwise indicated, the term "alkene" refers to an alkyl having C₂-C₂₄, for example, C₂-C₈ carbon atoms and one or more carbon-carbon double bonds. Examples of alkenes include, but are not limited to, vinyl, allyl, propenyl, butenyl, pentenyl, hexenyl and their branched isomers. Alkenes may be cyclic or polycyclic. Examples of cyclic alkenes include, but are not limited to, cyclopentenyl, cyclohexenyl. An alkene group can be unsubstituted or substituted with one or more suitable substituents selected among alkyl, alkylhalides, halogen atoms such as fluorine, chlorine, bromine, iodine, hydroxyl, alkoxy, nitro (-NO₂), nitrile (-CN), and aryl groups, with alkyl as defined previously and alkylhalides, alkoxy and aryl groups as defined hereinafter. Non-limiting examples of substituted alkene can be cinnamyl, 4-hydroxycinnamyl or coumaryl.

As used herein, and unless otherwise indicated, the term "alkyne" refers to an alkyl having C₂-C₁₂, for example, C₂-C₈ carbon atoms and one or more carbon-carbon triple bonds. Examples of alkynes include, but are not limited to acetylenyl, propynyl, butynyl, pentynyl, hexynyl and their branched isomers. An alkyne can be unsubstituted or substituted with one or more suitable substituents selected among halogen atoms such as fluorine, chlorine, bromine, iodine, hydroxyl, alkyl, alkylhalides, alkoxy, nitro (-NO₂), nitrile (-CN), and aryl, with alkyl as defined previously and alkoxy and aryl defined hereinafter.

As used herein, and unless otherwise indicated, the term "alkyl halide" refers to an alkyl as described above in which at least one hydrogen atom is substituted by a halogen atom selected from fluorine, chlorine, bromine and iodine. Non-limiting examples of alkyl halides are methyl fluoride, methyl chloride, methyl bromide, methyl iodide, ethyl fluoride, ethyl chloride, ethyl bromide, methyl difluoride, methyl dichloride, methyl chlorofluoride, methyl bromochlorofluoride, 2-chloropropyl, fluorocyclopentyl, (dibromomethyl)cyclohexyl, 2-iodo-2-methyl propyl, 2,4-dibromopentyl, methyl trifluoride, methyl trichloride, methyl tribromide, methyl triiodide.

As used herein, and unless otherwise indicated, the term "alkoxy" means an alkyl as defined above that is linked to another group via an oxygen atom (*i.e*. -O-alkyl).

As used herein, and unless otherwise indicated, the term "hydroxyl" means -OH.

As used herein, and unless otherwise indicated, the term "halogen or halide" employed or in combination with other terms means fluorine, chlorine, bromine, iodine.

As used herein, and unless otherwise indicated, the term "aryl" employed alone or in combination with other terms means an aromatic hydrocarbon having up to 14 carbon atoms, for example, 6 to 14 carbon atoms, which can be a single ring (monocyclic) or multiple rings (bicyclic, up to three rings) fused together or linked covalently. Any suitable ring position of the aryl moiety can be covalently linked to the defined chemical structure. Examples of aryl include but are not limited to phenyl, 1-naphtyl, 2-naphtyl, dihydronaphtyl, tetrahydronaphtyl, biphenyl, anthryl, phenanthryl. An aryl can be unsubstituted or substituted with one or more suitable substituents selected among halogen atoms such as fluorine, chlorine, bromine, iodine, hydroxyl, alkyl, alkyl halide, alkoxy, nitro (-NO₂), nitrile (-CN), -CO-aryl, -CO-alkyl, -CO-alkoxy, -CO-H, -CO-heteroaryl, sulfonamide (-SO₂-NR₃₅R₃₆) with R₃₅ and R₃₆ as defined hereafter for the term "amine or amino), -SO₃, and aryl, with alkyl, alkyl halide, alkoxy, aryl and heteroaryl as defined herein. Non-limiting examples of substituted aryl can be tolyl, methoxyphenyl, dimethoxy phenyl, trimethoxyphenyl, fluorophenyl, difluorophenyl, methyltrifluoride phenyl, nitrophenyl, methylnitrophenyl, methoxynitrophenyl, dimethoxynitrophenyl chloronitrophenyl, nitrilphenyl, tolylnitrophenyl, methoxynapthtyl, -CO-phenyl, -SO₂-NR₃₅R₃₆ with R₃₅ and R₃₆ being independently an alkyl such as methyl, ethyl, propyl, butyl, pentyl, hexyl.

As used herein, and unless otherwise indicated, the term "heteroaryl" means a 5 to 24, for example 5 to 10, membered mono- or polycyclic aromatic substituent where at least 2 atoms are carbon atoms and 1 to 4 atoms are heteroatoms independently selected among nitrogen, oxygen or sulfur. When the heteroaryl is polycyclic, for example bicyclic, the rings may be fused together. Non limiting examples of heteroaryl include, but are not limited to, furyl, benzofuranyl, pyrrolyl, indolyl, isoindolyl, azaindolyl, thiophenyl, benzothiophenyl, 2-pyridyl, 3-pyridyl, 4-pyridyl, 2-quinolinyl, 1*H*-1,2,3-triazolyl, 2*H-*1,2,3-triazolyl, 1*H*-1,2,4-triazolyl, 4*H*-1,2,4-triazolyl, isoquinolyl, imidazolyl, benzimidazolyl, indolizinyl, pyrazolyl, oxazolyl, isoxazolyl, benzoxazolyl, thiazolyl, benzothiazolyl, isothiazolyl, pyridazinyl, pyrimidinyl, pyrazinyl, triazinyl, cinnolinyl, phtalazinyl, quinazolinyl, chromenonyl, coumarinyl, quinolonyl. The heteroaryl group can be substituted by one or more alkoxy; one or more hydroxyl; one or more aryl; one or more halogen atoms; one or more nitro (-NO₂); one or more nitrile (-CN); one or more alkyl groups; one or more alkyl halide, where alkyl, alkoxy and aryl are defined as in the present invention. Non-limiting examples of heteroaryl can be 1*H*-1,2,3-triazolyl, 2*H-*1,2,3-triazolyl, 1*H*-1,2,4-triazolyl, 4*H*-1,2,4-triazolyl, 2-pyridyl, 3-pyridyl, 4-pyridyl, thiophenyl, benzofuranyl, 2-chromenonyl, 4-chromenonyl, 3-coumarinyl, 4-coumarinyl, 4-quinolonyl, 2-quinolonyl said heteroaryls being optionally substituted by an alkyl. Other non limiting examples of substituted heteroaryls are methyl-, ethyl-benzothiophenyl; methyl-, methoxy-benzofuranyl; methyl-pyrrolyl; methylindolyl.

As used herein, and unless otherwise indicated, the term "heterocycle" means a 5 to 24, for example, 5 to 10 membered, mono- or polycyclic substituent, saturated or unsaturated (nonaromatic), having 1 to 4 heteroatoms, independently selected among nitrogen, oxygen or sulfur. When the heterocycle is polycyclic, for example bicyclic, the rings may be fused together. Non limiting examples of heterocycle groups include the morpholinyl, piperidinyl, piperazinyl, pyrrolidinyl, imidazolidinyl, imidazolinyl, pyrazolidinyl, tetrahydrofuranyl, tetrahydropyranyl, thianyl, oxazolidinyl, isoxazolidinyl, thiazolidinyl and isothiazolidinyl substituents. Also included in the definition of heterocycle are moieties that have one or more (*i.e.* two) aromatic rings fused (*i.e.* having a bond in common with) to the nonaromatic heterocycle ring, for example, phtalimidyl, indolinyl. The heterocycle can optionally substituted by one or more substituents selected among hydroxyl alkoxy groups; halogen atoms; nitro groups; nitrile groups; aryl groups optionally substituted; alkyl groups; alkyl halide, where alkyl, alkyl halide and alkoxy and aryl groups are as defined herein.

As used herein, and unless otherwise indicated, an alkaline cation means cations of lithium (Li⁺), sodium (Na⁺), potassium (K⁺), rubidium (Rb⁺), or cesium (Cs⁺).

As used herein, and unless otherwise indicated, "amine or amino" means a group of formula -NR₃₅R₃₆, where
- R₃₅ and R₃₆ are independently, a hydrogen atom, an alkyl group, an alkene group, an alkyne group, an aryl group, an heteroaryl group, a heterocycle, said alkyl, alkene, alkyne, aryl, heteroaryl, and heterocycle being optionally substituted, or
- R₃₅ and R₃₆ form together with the nitrogen atom to which they are linked à heteroaryl or a heterocycle, said heteroaryl and heterocycle being optionally substituted.

As used herein, and unless otherwise indicated, an agrochemical is a chemical product used in agriculture. In most cases, this term refers to pesticides including insecticides, herbicides, fungicides and nematicides. It may also include synthetic fertilizers, hormones and other chemical growth agents, and concentrated stores of raw animal manure.

The synthesis of labeled carboxyl compounds of formula (I) in the present process is one-pot. In the context of the invention, a "one-pot" synthesis is a synthesis whereby a reactant is subjected to successive chemical reactions in just one reactor without isolating the intermediate compounds formed in the reactor.

It is of note that in the literature only sequential multistep carbon isotope insertions are known. One of the significant advantages of the present process is that the synthesis of labeled carboxylic compounds of formula (I) does not require more than one step.

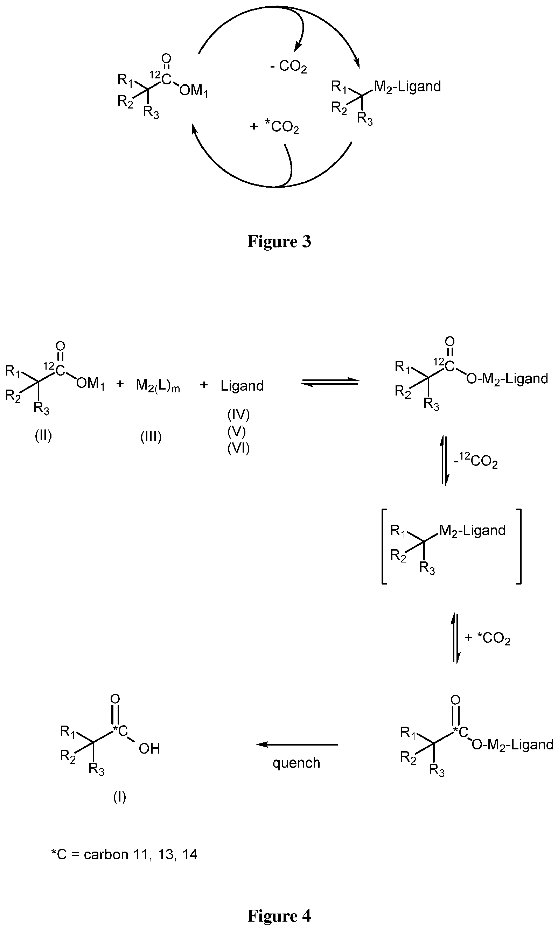

While not wishing to be bound by theory, the choice of the compounds, reactants and the reaction conditions and more specifically the catalyst system in the process of the invention allows a one-pot one step synthesis of carbon labeled organic compounds containing a carbon labeled carboxyl group. The process of the invention actually involves a direct insertion of carbon-11(¹¹C), carbon-13(¹³C), and carbon-14(¹⁴C), via a carbon-12 (¹²C)/carbon-11(¹¹C) a carbon-12 (¹²C)/carbon-13(¹³C) or a carbon-12 (¹²C)/carbon-14(¹⁴C) isotope exchange at the carbon atom of the carboxyl function in one step. Still not wishing to be bound by theory, the isotope exchange at the carbon atom of the carboxyl function in one step is the result of a dynamic decarboxylation/carboxylation reaction. The catalyst system used in the process of the invention is able to reversibly decarboxylate and re-carboxylate the carboxylic moiety. This is illustrated in Figures 3 and 4. Current processes are not reversible: the decarboxylation of carboxylic acid yields a reactive carbon nucleophile which is further trapped by an electrophile in a coupling reaction.

Still not wishing to be bound by theory, as shown in Figures 3 and 4, the process of the invention enables a carbon isotope exchange in only one-step by means of a dynamic decarboxylation/carboxylation procedure. The transformation takes place by the following mechanism: at first a metal catalyzed decarboxylation of the metal-ligand coordinated carboxylate generates a metal-ligand intermediate. Under the suitable reaction conditions the metal-ligand intermediate will carboxylate in presence of labeled *CO₂ to form the labeled metal-ligand coordinated carboxylate, which upon quenching yields the labeled compound of formula (I).

As already mentioned and not wishing to be bound by theory, it seems that the catalyst system plays an important role in the reversible decarboxylation/carboxylation of the carboxyl containing organic compound.

The catalyst system of the invention comprises an inorganic salt of formula (III)

M₂(L)ₘ (III)

wherein
- M₂ is a transition metal selected from Cu, Pd, Ni, Ru, Ag, Rh, Fe, Co, Zn, Ir, Au, Pt,
- m is 1 or 2;
- L is a halogen atom selected from fluorine, chlorine, bromine, and iodide, a triflate or trifluoromethylsulfonate, a tosylate or p-toluenesulfonate, a mesylate or methanesulfonate, -CN,
and a ligand of formula (IV), formula (V) or formula (VI).

In a **first** embodiment, provided herein is a process for the synthesis of a compound containing a carbon labeled carboxyl group according to formula (I) 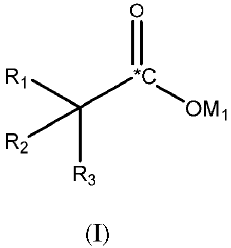 wherein
▪ *C is a ¹¹C, ¹³C or ¹⁴C isotope;
▪ R₁, R₂ and R₃ are, independently, a hydrogen atom, an aryl, a heteroaryl, a heterocycle, an alkyl, an alkyl halide, an alkene or an alkyne, said aryl, heteroaryl, heterocycle, alkene, alkyne and alkyl groups being optionally substituted, or R₁ and R₂ form together with the carbon atom to which they are linked a carbonyl (-(C=O)- and R₃ is as defined above, or
   R₁ and R₂ form together with the carbon atom to which they are linked an alkene having at least one double bond, with one double bond being alpha to the carboxyl group, said alkene being optionally substituted, and R₃ is as defined above, or R₁, R₂ and R₃ form together with the carbon atom to which they are linked an aryl, a heteroaryl, or a heterocycle, said aryl, heteroaryl and heterocycle being optionally substituted;
▪ M₁ is a hydrogen atom, a silver cation (Ag⁺), an alkaline cation selected from lithium (Li⁺), sodium (Na⁺), potassium (K⁺), rubidium (Rb⁺), or cesium (Cs⁺);
characterized in that
- an organic compound containing a carboxyl group according to formula (II) 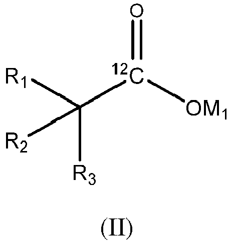 wherein R₁, R₂, R₃ and M₁ are as defined above,
- is reacted with a labeled *CO₂ wherein *C is an isotope as defined above,
- in the presence of a catalyst system comprising an inorganic salt of formula (III)

   M₂(L)ₘ (III)

   wherein
   - M₂ is a transition metal selected from Cu, Pd, Ni, Ru, Ag, Rh, Fe, Co, Zn, Ir, Au, Pt,
   - m is 1 or 2;
   - L is a halogen atom selected from fluorine, chlorine, bromine, and iodide, a triflate or trifluoromethylsulfonate, a tosylate or p-toluenesulfonate, a mesylate or methanesulfonate, -CN,
   and a ligand of formula (IV) 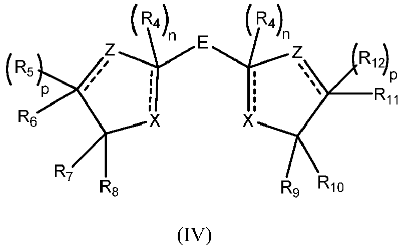 wherein
   - n is 0 or 1;
   - n' is 0 or 1;
   - n" is 0 or 1;
   - p is 0 or 1;
   - E is a single bond, 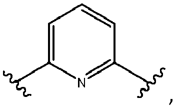 -C(R₁₃R₁₄)- with R₁₃ and R₁₄, independently being a hydrogen atom, an alkyl, an aryl, -CN, -NO₂, a halogen atom selected from F, Cl, Br, I;
   - X is N(R₁₅)_{n'}, O, P, S(R₁₅)_{n'}, or P(R₁₅)_{n'} with R₁₅ being a hydrogen atom or an alkyl and with the proviso that when X is N(R₁₅)_{n'}, S(R₁₅)_{n'}, or P(R₁₅)_{n'}, and n'=n=0, ---- is a double bond;
   - Z is N(R₁₅)_{n"}, O, P, S(R₁₅)_{n"}, or P(R₁₅)_{n"}, with R₁₅ being a hydrogen atom or an alkyl and with the proviso that when Z is N(R₁₅)_{n"}, S(R₁₅)_{n"}, or P(R₁₅)_{n"}, and n"=p=0, ---- is a double bond;
   - R₄, R₅, R₆, R₇, R₈, R₉, R₁₀, R₁₁ and R₁₂ are, independently, a hydrogen atom, an alkyl, an alkoxy, an aryl, or -CN, said alkyl and aryl being optionally substituted, or
      R₄, R₅, R₈, R₉, and R₁₂ are, independently, a hydrogen atom, an alkyl, an alkoxy, an aryl, or -CN, R₆ and R₇ and/or R₁₀ and R₁₁ form together with the carbon atoms to which they are linked a heterocycle, said alkyl, aryl and heterocycle being optionally substituted.

In an embodiment of the first embodiment of the invention, in the ligand of formula (IV) 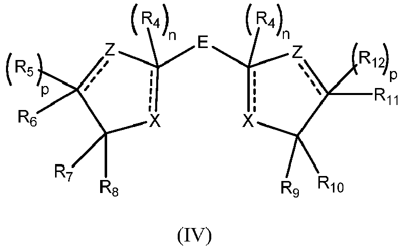
- p is 0 or 1;
- E is a single bond, 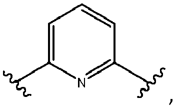 -C(R₁₃R₁₄)- with R₁₃ and R₁₄, independently being a hydrogen atom, a C₁-C₈ alkyl, or -CN;
- X is N(R₁₅)_{n'}, n' = n = 0, and ---- is a double bond;
- Z is O;
- R₅, R₆, R₇, R₈, R₉, R₁₀, R₁₁ and R₁₂ are, independently, a hydrogen atom, a C₁-C₈ alkyl, an aryl having 6 to 14 carbon atoms, or a -CN, said alkyl and aryl being optionally substituted, or
   R₅, R₈, R₉, and R₁₂ are, independently, a hydrogen atom, a C₁-C₈ alkyl, an aryl having 6 to 14 carbon atoms, or -CN, R₆ and R₇ and/or R₁₀ and R₁₁ form together with the carbon atoms to which they are linked a 5 to 10 membered heterocycle, said alkyl, aryl and heterocycle being optionally substituted.

In an embodiment of the first embodiment, in the ligand of formula (IV),
- p is 1;
- E is a single bond; 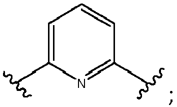 -C(R₁₃R₁₄)- with R₁₃ and R₁₄, independently being a hydrogen atom, a methyl, ethyl, propyl, butyl, pentyl, hexyl, octyl, and their branched isomers such as isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1 -butyl, 3,3-dimethyl-1-butyl, 2-ethyl-1-butyl, isobutyl, t-butyl, isopentyl, neopentyl, cyclopropyl, cyclobutyl, cyclopentyl, cyclohexyl; or -CN;
- X is N(R₁₅)_{n'}, n' = n = 0, and ---- is a double bond;
- Z is O;
- R₅, R₆, R₇, R₈, R₉, R₁₀, R₁₁ and R₁₂ are, independently, a hydrogen atom; a methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl, cyclopropyl, cyclobutyl, cyclopentyl, cyclohexyl, benzyl; a phenyl, a phenyl substituted by a methyl, ethyl, propyl, butyl, isopropyl, isobutyl, t-butyl; or
   R₅, R₈, R₉, and R₁₂ are, independently, a hydrogen atom; a methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl, cyclopropyl, cyclobutyl, cyclopentyl, cyclohexyl, benzyl; a phenyl, a phenyl substituted by a methyl, ethyl, propyl, butyl, isopropyl, isobutyl, t-butyl; R₆ and R₇ and/or R₁₀ and R₁₁ form together with the carbon atoms to which they are linked phtalimidyl, indolinyl, 1,2-hydrindenyl optionally substituted by a methyl, ethyl, propyl, butyl, isopropyl, isobutyl, t-butyl.

In a preferred embodiment of the first embodiment, the ligand of formula (IV) is,

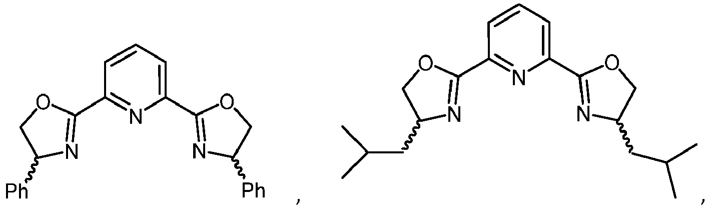

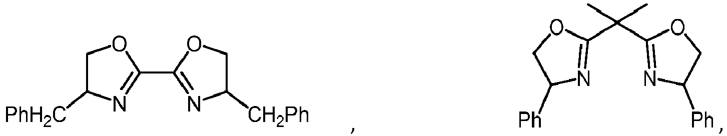

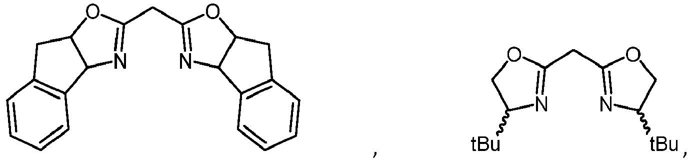

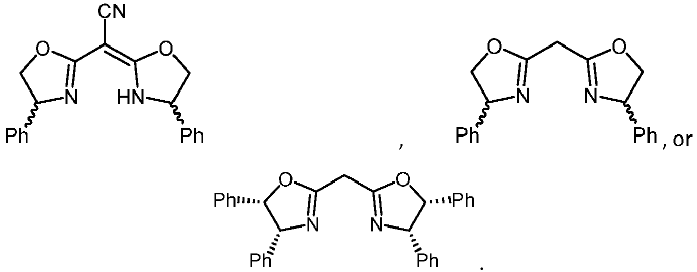

In a **second** embodiment, provided herein is a process for the synthesis of a carbon labeled organic compound containing a carbon labeled carboxyl group according to formula (I) 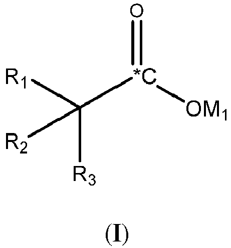 wherein
▪ *C is a ¹¹C, ¹³C or ¹⁴C isotope;
▪ R₁, R₂ and R₃ are, independently, a hydrogen atom, an aryl, a heteroaryl, a heterocycle, an alkyl, an alkyl halide, an alkene or an alkyne, said aryl, heteroaryl, heterocycle, alkene, alkyne and alkyl groups being optionally substituted, or R₁ and R₂ form together with the carbon atom to which they are linked a carbonyl (-(C=O)- and R₃ is as defined above, or
   R₁ and R₂ form together with the carbon atom to which they are linked an alkene having at least one double bond, with one double bond being alpha to the carboxyl group, said alkene being optionally substituted, and R₃ is as defined above, or R₁, R₂ and R₃ form together with the carbon atom to which they are linked an aryl, a heteroaryl, or a heterocycle, said aryl, heteroaryl and heterocycle being optionally substituted;
▪ M₁ is a hydrogen atom, a silver cation (Ag⁺), an alkaline cation selected from lithium (Li⁺), sodium (Na⁺), potassium (K⁺), rubidium (Rb⁺), or cesium (Cs⁺);
characterized in that
- an organic compound containing a carboxyl group according to formula (II) 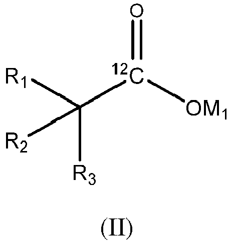 wherein R₁, R₂, R₃ and M₁ are as defined above,
- is reacted with a labeled *CO₂ wherein *C is an isotope as defined above,
- in the presence of a catalyst system comprising an inorganic salt of formula (III)

   M₂(L)ₘ (III)

   wherein
   - M₂ is a transition metal selected from Cu, Pd, Ni, Ru, Ag, Rh, Fe, Co, Zn, Ir, Au, Pt,
   - m is 1 or 2;
   - L is a halogen atom selected from fluorine, chlorine, bromine, and iodide, a triflate or trifluoromethylsulfonate, a tosylate or p-toluenesulfonate, a mesylate or methanesulfonate, -CN,
   and a ligand of formula (V) 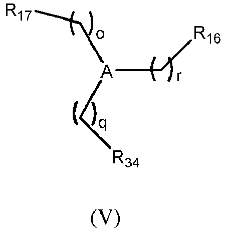 wherein
   - o is 0 or 1;
   - q is 0 or 1;
   - r is 0 or 1;
   - t is 0, 1, 2 or 3;
   - A is N or P;
   - R₁₆, R₁₇ and R₃₄ are, independently, a hydrogen atom, an alkyl, an alkoxy, an aryl, a heteroaryl, a heterocycle, said alkyl, aryl, heteroaryl and heterocycle being optionally substituted, -(C=S)-NR₃₅R₃₆, -(C=O)-NR₃₅R₃₆, -(CH₂)ₜ-NR₃₅R₃₆, -(CH₂)ₜ-PR₃₅R₃₆ with
      R₃₅ and R₃₆ being independently, a hydrogen atom, an alkyl, an alkene, an alkyne, an aryl, a heteroaryl, a heterocycle, said alkyl, alkene, alkyne, aryl, heteroaryl and heterocycle being optionally substituted,
      R₃₅ and R₃₆ form together with the nitrogen atom to which they are linked an optionally substituted heteroaryl or heterocycle.

In an embodiment of the second embodiment, in the ligand of formula (V)
- o is 0 or 1;
- q is 0 or 1;
- r is 0 or 1;
- t is 0, 1, 2 or 3;
- A is N or P;
- R₁₆, R₁₇ and R₃₄ are, independently, a hydrogen atom, a C₁-C₈ alkyl, an aryl having 6 to 14 carbon atoms, a 5 to 10 membered heteroaryl, a 5 to 10 membered heterocycle, said alkyl, aryl, heteroaryl and heterocycle being optionally substituted, -(C=S)-NR₃₅R₃₆, -(C=O)-NR₃₅R₃₆, -(CH₂)ₜ-NR₃₅R₃₆, -(CH₂)ₜ-PR₃₅R₃₆ with
   R₃₅ and R₃₆ being independently, a hydrogen atom, a C₁-C₈ alkyl, an aryl having 6 to 14 carbon atoms, a 5 to 10 membered heteroaryl, said alkyl, aryl, and heteroaryl being optionally substituted.

In an embodiment of the second embodiment, in the ligand of formula (V)
- o is 0 or 1;
- q is 0 or 1;
- r is 0 or 1;
- t is 0, 1, or 2;
- A is N or P;
- R₁₆, R₁₇ and R₃₄ are, independently, a hydrogen atom; a methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1 -butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl; a phenyl, methoxyphenyl, fluorophenyl, tolyl, trimethoxyphenyl; a 1*H*-1,2,3-triazolyl, 2*H*-1,2,3-triazolyl, 1*H*-1,2,4-triazolyl, 4*H-*1,2,4-triazolyl, 2-pyridyl, 3-pyridyl, 4-pyridyl, said heteroaryls being substituted by a C₁-C₈ alkyl selected from methyl, ethyl, propyl, butyl, isopropyl, isobutyl, t-butyl; -(C=S)-NR₃₅R₃₆, -(C=O)-NR₃₅R₃₆, -(CH₂)ₜ-NR₃₅R₃₆, -(CH₂)ₜ-PR₃₅R₃₆ with R₃₅ and R₃₆ being independently, a hydrogen atom; a methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl; a phenyl methoxyphenyl, fluorophenyl, tolyl, trimethoxyphenyl; a 1*H*-1,2,3-triazolyl, 2*H*-1,2,3-triazolyl, 1*H*-1,2,4-triazolyl, 4*H-*1,2,4-triazolyl, 2-pyridyl, 3-pyridyl, 4-pyridyl, said heteroaryls being substituted by a C₁-C₈ alkyl selected from methyl, ethyl, propyl, butyl, isopropyl, isobutyl, t-butyl.

In a preferred embodiment of the second embodiment, the ligand of formula (V) is, 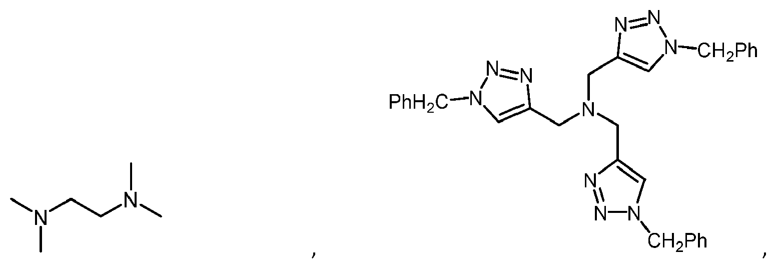 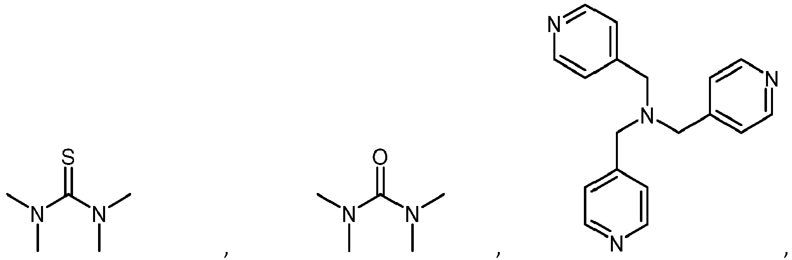 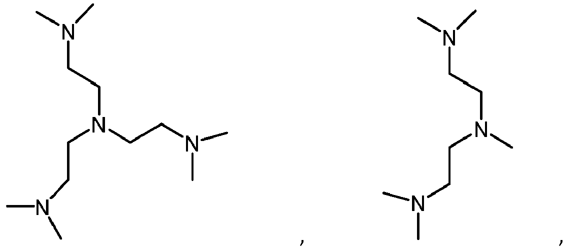 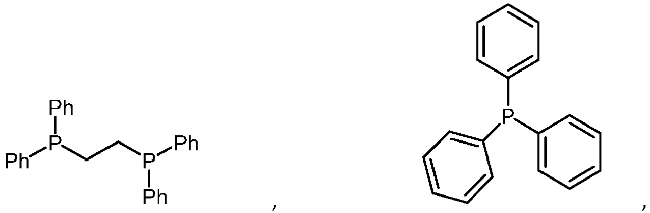 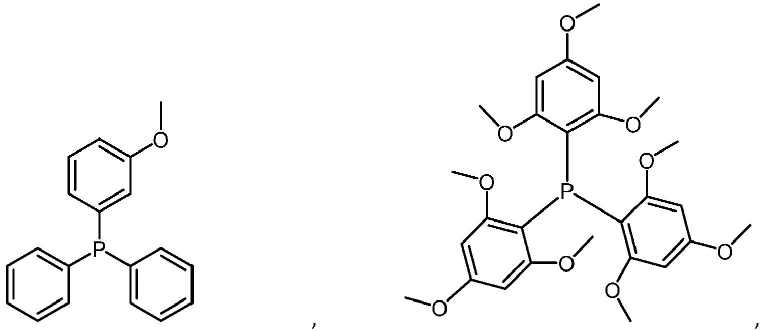 or

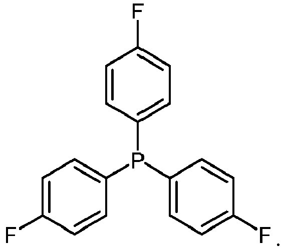

In a **third** embodiment, provided herein is a process for the synthesis of a carbon labeled organic compound containing a carbon labeled carboxyl group according to formula (I) 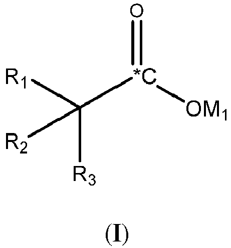 wherein
▪ *C is a ¹¹C, ¹³C or ¹⁴C isotope;
▪ R₁, R₂ and R₃ are, independently, a hydrogen atom, an aryl, a heteroaryl, a heterocycle, an alkyl, an alkyl halide, an alkene or an alkyne, said aryl, heteroaryl, heterocycle, alkene, alkyne and alkyl groups being optionally substituted, or
   R₁ and R₂ form together with the carbon atom to which they are linked a carbonyl (-(C=O)- and R₃ is as defined above, or
   R₁ and R₂ form together with the carbon atom to which they are linked an alkene having at least one double bond, with one double bond being alpha to the carboxyl group, said alkene being optionally substituted, and R₃ is as defined above, or
   R₁, R₂ and R₃ form together with the carbon atom to which they are linked an aryl, a heteroaryl, or a heterocycle, said aryl, heteroaryl and heterocycle being optionally substituted;
▪ M₁ is a hydrogen atom, a silver cation (Ag⁺), an alkaline cation selected from lithium (Li⁺), sodium (Na⁺), potassium (K⁺), rubidium (Rb⁺), or cesium (Cs⁺);
characterized in that
- an organic compound containing a carboxyl group according to formula (II) 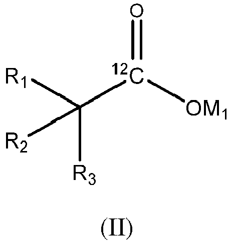 wherein R₁, R₂, R₃ and M₁ are as defined above,

- is reacted with a labeled *CO₂ wherein *C is an isotope as defined above,
- in the presence of a catalyst system comprising an inorganic salt of formula (III)

   M₂(L)ₘ (III)

   wherein
   - M₂ is a transition metal selected from Cu, Pd, Ni, Ru, Ag, Rh, Fe, Co, Zn, Ir, Au, Pt,
   - m is 1 or 2;
   - L is a halogen atom selected from fluorine, chlorine, bromine, and iodide, a triflate or trifluoromethylsulfonate, a tosylate or p-toluenesulfonate, a mesylate or methanesulfonate, -CN,
   and a ligand of formula (VI) 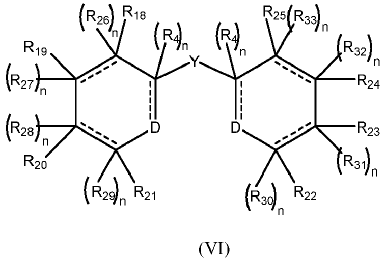 wherein
   - n is 0 or 1;
   - n'" is 0 or 1;
   - Y is a single bond, 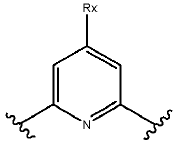 with Rₓ being a hydrogen atom or an alkyl;
   - D is N(R₁₅)_{n'"}, O, P, S(R₁₅)_{n"'}, or P(R₁₅)_{n'"} with R₁₅ being a hydrogen atom or an alkyl and with the proviso that when D is N(R₁₅)_{n'"}, S(R₁₅)_{n'"}, or P(R₁₅)_{n'"} and n'"=n=0, ---- is a double bond;
   - R₄ is a hydrogen atom, an alkyl, an alkoxy, an aryl, or -CN, said alkyl and aryl being optionally substituted;
   - R₁₈, R₁₉, R₂₀, R₂₁, R₂₂, R₂₃, R₂₄, R₂₅, R₂₆, R₂₇, R₂₈, R₂₉, R₃₀, R₃₁, R₃₂ and R₃₃ are, independently, a hydrogen atom, an alkyl, an alkoxy, an aryl, or a -CN, said alkyl and aryl being optionally substituted, or
      when Y is a single bond, n=0, ---- is a double bond, R₁₈, R₂₁, R₂₂, R₂₅, R₂₆, R₂₇, R₂₈, R₂₉, R₃₀, R₃₁, R₃₂ and R₃₃ are, independently, a hydrogen atom, an alkyl, an alkoxy, an aryl, or -CN, and R₁₉ and R₂₀ and/or R₂₃ and R₂₄ form together with the carbon atoms to which they are linked an aryl, said alkyl and aryl being optionally substituted.

In an embodiment of the third embodiment, in the ligand of formula (VI)
- n is 0 or 1;
- n'" is 0 or 1;
- Y is a single bond, 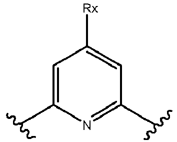 with Rₓ being a hydrogen atom or a C₁-C₈ alkyl;
- D is N(R₁₅)_{n"'}, O, P, S(R₁₅)_{n"'}, or P(R₁₅)_{n"'} with R₁₅ being a hydrogen atom or a C₁-C₈ alkyl and with the proviso that when D is N(R₁₅)_{n'"}, S(R₁₅)_{n'"}, or P(R₁₅)_{n'"} and n'"=n=0, ---- is a double bond;
- R₄ is a hydrogen atom, a C₁-C₈ alkyl, a C₁-C₈ alkoxy, an aryl having 6 to 14 carbon atoms, or -CN, said alkyl and aryl being optionally substituted;
- R₁₈, R₁₉, R₂₀, R₂₁, R₂₂, R₂₃, R₂₄, R₂₅, R₂₆, R₂₇, R₂₈, R₂₉, R₃₀, R₃₁, R₃₂ and R₃₃ are, independently, a hydrogen atom, a C₁-C₈ alkyl, a C₁-C₈ alkoxy, an aryl having 6 to 14 carbon atoms, or a -CN, said alkyl and aryl being optionally substituted, or when Y is a single bond, n=0, ---- is a double bond, R₁₈, R₂₁, R₂₂, R₂₅, R₂₆, R₂₇, R₂₈, R₂₉, R₃₀, R₃₁, R₃₂ and R₃₃ are, independently, a hydrogen atom, a C₁-C₈ alkyl, an C₁-C₈ alkoxy, an aryl having 6 to 14 carbon atoms, or -CN, and R₁₉ and R₂₀ and/or R₂₃ and R₂₄ form together with the carbon atoms to which they are linked an aryl having 6 to 14 carbon atoms, said alkyl and aryl being optionally substituted.

In an embodiment of the third embodiment, in the ligand of formula (VI)
- n is 0 or 1;
- n'" is 0 or 1;
- Y is a single bond; 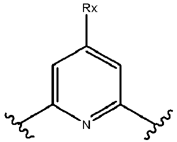 with Rₓ being a hydrogen atom or a methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1 -butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl;
- D is N(R₁₅)_{n"'}, O, P, S(R₁₅)_{n'"}, or P(R₁₅)_{n'"} with R₁₅ being a hydrogen atom; a methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-lpropyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl; and with the proviso that when D is N(R₁₅)_{n"'}, S(R₁₅)_{n"'}, or P(R₁₅)_{n"'} and n"'=n=0, ---- is a double bond;
- R₄ is a hydrogen atom; a methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl; a methyloxy, ethyloxy, propyloxy, butyloxy, pentyloxy, hexyloxy, octyloxy, isopropyloxy, 2-methyl-1-propyloxy, 2-methyl-2-propyloxy, 2-methyl-1-butyloxy, 3-methyl-1-butyloxy, 2-methyl-3-butyloxy, 2,2-dimethyl-1propyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2,2-dimethyl-1-butyloxy, 3,3-dimethyl-1-butyloxy, 2-ethyl-1-butyloxy, isobutyloxy, t-butyloxy, isopentyloxy, neopentyloxy, benzyloxy, cyclopropyloxy, cyclobutyloxy, cyclopentyloxy, cyclohexyloxy; a phenyl, methoxyphenyl, fluorophenyl, tolyl, trimethoxyphenyl; or -CN;
- R₁₈, R₁₉, R₂₀, R₂₁, R₂₂, R₂₃, R₂₄, R₂₅, R₂₆, R₂₇, R₂₈, R₂₉, R₃₀, R₃₁, R₃₂ and R₃₃ are, independently, a hydrogen atom; a methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl; a methyloxy, ethyloxy, propyloxy, butyloxy, pentyloxy, hexyloxy, octyloxy, isopropyloxy, 2-methyl-1-propyloxy, 2-methyl-2-propyloxy, 2-methyl-1-butyloxy, 3-methyl-1-butyloxy, 2-methyl-3-butyloxy, 2,2-dimethyl-1propyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2,2-dimethyl-1-butyloxy, 3,3-dimethyl-1-butyloxy, 2-ethyl-1-butyloxy, isobutyloxy, t-butyloxy, isopentyloxy, neopentyloxy, benzyloxy, cyclopropyloxy, cyclobutyloxy, cyclopentyloxy, cyclohexyloxy; a phenyl, methoxyphenyl, fluorophenyl, tolyl, trimethoxyphenyl; or a -CN, or
   when Y is a single bond, n=0, ---- is a double bond, R₁₈, R₂₁, R₂₂, R₂₅, R₂₆, R₂₇, R₂₈, R₂₉, R₃₀, R₃₁, R₃₂ and R₃₃ are, independently, a hydrogen atom; a methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1 -butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl; a methyloxy, ethyloxy, propyloxy, butyloxy, pentyloxy, hexyloxy, octyloxy, isopropyloxy, 2-methyl-1-propyloxy, 2-methyl-2-propyloxy, 2-methyl-1-butyloxy, 3-methyl-1-butyloxy, 2-methyl-3-butyloxy, 2,2-dimethyl-1propyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2,2-dimethyl-1-butyloxy, 3,3-dimethyl-1-butyloxy, 2-ethyl-1-butyloxy, isobutyloxy, t-butyloxy, isopentyloxy, neopentyloxy, benzyloxy, cyclopropyloxy, cyclobutyloxy, cyclopentyloxy, cyclohexyloxy; a phenyl, methoxyphenyl, fluorophenyl, tolyl, trimethoxyphenyl; or -CN, and R₁₉ and R₂₀ and/or R₂₃ and R₂₄ form together with the carbon atoms to which they are linked a phenyl, methoxyphenyl, fluorophenyl, tolyl, trimethoxyphenyl.

In a preferred embodiment of the third embodiment the ligand of formula (VI) is

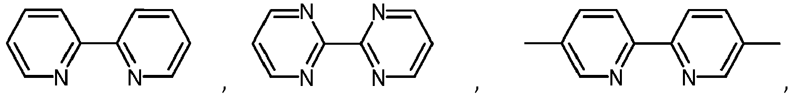

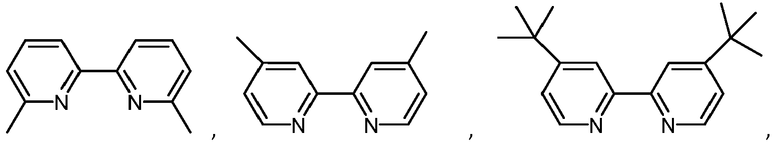

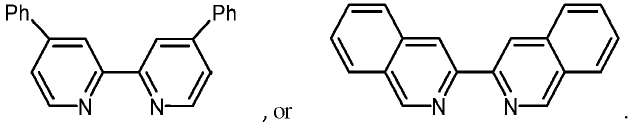

In a **fourth** embodiment, the catalyst system of the invention comprises, in addition to the ligands of formula (IV), formula (V) or formula (VI) according to the first to third embodiments, an inorganic salt of formula (III)

M₂(L)ₘ (III)

wherein
- M₂ is a transition metal selected from Cu, Pd, Ni, Ru, Ag, Rh, Fe, Co, Zn, Ir, Au, Pt,
- m is 1 or 2;
- L is a halogen atom selected from fluorine, chlorine, bromine, and iodide, a triflate or trifluoromethylsulfonate, a tosylate or *p*-toluenesulfonate, a mesylate or methanesulfonate, -CN.

In an embodiment of the fourth embodiment, in the inorganic salt of formula (III)

M₂(L)ₘ (III)

- M₂ is a transition metal selected from Cu, Pd, Ni, Ru, Ag, Rh, Fe, Co, Zn, Ir, Au, Pt,
- m is 1;
- L is a halogen atom selected from chlorine, bromine, and iodide, a triflate or trifluoromethylsulfonate, a tosylate or *p*-toluenesulfonate, a mesylate or methanesulfonate, -CN.

In a preferred embodiment of the fourth embodiment, in the inorganic salt of formula (III)

M₂(L)ₘ (III)

- M₂ is a transition metal selected from Cu, Ag, Pd, Ni, Au,
- m is 1;
- L is a halogen atom selected from chlorine, bromine, and iodide, a triflate or trifluoromethylsulfonate, -CN.

The present invention provides a process for the synthesis of a carbon labeled organic compound containing a carbon labeled carboxyl group according to formula (I) 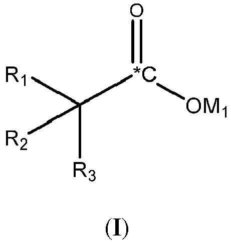 wherein
▪ *C is a ¹¹C, ¹³C or ¹⁴C isotope;
▪ R₁, R₂ and R₃ are, independently, a hydrogen atom, an aryl, a heteroaryl, a heterocycle, an alkyl, an alkyl halide, an alkene or an alkyne, said aryl, heteroaryl, heterocycle, alkene, alkyne and alkyl groups being optionally substituted, or
   R₁ and R₂ form together with the carbon atom to which they are linked a carbonyl (-(C=O)- and R₃ is as defined above, or
   R₁ and R₂ form together with the carbon atom to which they are linked an alkene having at least one double bond, with one double bond being alpha to the carboxyl group, said alkene being optionally substituted, and R₃ is as defined above, or
   R₁, R₂ and R₃ form together with the carbon atom to which they are linked an aryl, a heteroaryl, or a heterocycle, said aryl, heteroaryl and heterocycle being optionally substituted;
▪ M₁ is a hydrogen atom, a silver cation (Ag⁺), an alkaline cation selected from lithium (Li⁺), sodium (Na⁺), potassium (K⁺), rubidium (Rb⁺), or cesium (Cs⁺);
characterized in that
- an organic compound containing a carboxyl group according to formula (II) 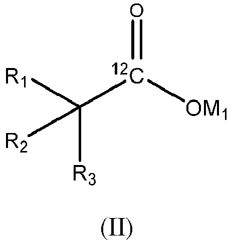 wherein R₁, R₂, R₃ and M₁ are as defined above,
- is reacted with a labeled *CO₂ wherein *C is an isotope as defined above,
- in the presence of a catalyst system according to the first, second, third and fourth embodiments of the present invention.

In **fifth** embodiment of the invention, the process of the invention provides a carbon labeled organic compound containing a carbon labeled carboxyl group of formula (I) and an organic compound containing a carboxyl group of formula (II), wherein
R₁, R₂ and R₃ are, independently, a hydrogen atom, an aryl having 6 to 14 carbon atoms, a 5 to 10 membered heteroaryl, a C₁-C₈ alkyl, a C₁-C₈ alkyl halide, said aryl, heteroaryl and alkyl groups being optionally substituted.

In this fifth embodiment in the carbon labeled organic compound containing a carbon labeled carboxyl group of formula (I) and the organic compound containing a carboxyl group of formula (II), R₁, R₂ and R₃ are, independently,
- a hydrogen atom,
- a phenyl, 1-naphtyl, 2-naphtyl, dihydronaphtyl, tetrahydronaphtyl, biphenyl, anthryl, phenanthryl, all optionally substituted with one or more substituents selected among hydroxyl, methyl fluoride, methyl chloride, methyl bromide, methyl iodide, ethyl fluoride, ethyl chloride, ethyl bromide, methyl difluoride, methyl dichloride, methyl chlorofluoride, methyl bromochlorofluoride, 2-chloropropyl, fluorocyclopentyl, (dibromomethyl)cyclohexyl, 2-iodo-2-methyl propyl, 2,4-dibromopentyl, methyl trifluoride, methyl trichloride, methyl tribromide, methyl triiodide; fluorine, chlorine, bromine, iodine; a methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1 -butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl; a methyloxy, ethyloxy, propyloxy, butyloxy, pentyloxy, hexyloxy, octyloxy, isopropyloxy, 2-methyl-1-propyloxy, 2-methyl-2-propyloxy, 2-methyl-1-butyloxy, 3-methyl-1-butyloxy, 2-methyl-3-butyloxy, 2,2-dimethyl-1propyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2,2-dimethyl-1-butyloxy, 3,3-dimethyl-1-butyloxy, 2-ethyl-1-butyloxy, isobutyloxy, t-butyloxy, isopentyloxy, neopentyloxy, benzyloxy, cyclopropyloxy, cyclobutyloxy, cyclopentyloxy, cyclohexyloxy; nitro (-NO₂); nitrile (-CN); -CO-phenyl;
- a methyl fluoride, a methyl chloride, a methyl bromide, a methyl iodide, an ethyl fluoride, an ethyl chloride, an ethyl bromide, a methyl difluoride, a methyl dichloride, a methyl chlorofluoride, a methyl bromochlorofluoride, a 2-chloropropyl, a fluorocyclopentyl, a (dibromomethyl)cyclohexyl, a 2-iodo-2-methyl propyl, a 2,4-dibromopentyl, a methyl trifluoride, a methyl trichloride, a methyl tribromide, a methyl triiodide;
- a methyl, an ethyl, a propyl, a butyl, a pentyl, a hexyl, an octyl, an isopropyl, a 2-methyl-1-propyl, a 2-methyl-2-propyl, a 2-methyl-1-butyl, a 3-methyl-1-butyl, a 2-methyl-3-butyl, a 2,2-dimethyl-1-propyl, a 2-methyl-1-pentyl, a 3-methyl-1-pentyl, a 4-methyl-1-pentyl, a 2-methyl-2-pentyl, a 3-methyl-2-pentyl, a 4-methyl-2-pentyl, a 2,2-dimethyl-1-butyl, a 3,3-dimethyl-1-butyl, a 2-ethyl-1-butyl, an isobutyl, a t-butyl, an isopentyl, a neopentyl, a cyclopropyl, a cyclobutyl, a cyclopentyl, a cyclohexyl;
- a furyl, a benzofuranyl, a pyrrolyl, an indolyl, an isoindolyl, an azaindolyl, a thiophenyl, a benzothiophenyl, a 2-pyridyl, a 3-pyridyl, a 4-pyridyl, a 2-quinolinyl, a 1*H*-1,2,3-triazolyl, a 2*H*-1,2,3-triazolyl, a 1*H*-1,2,4-triazolyl, a 4*H*-1,2,4-triazolyl, an isoquinolyl, an imidazolyl, a benzimidazolyl, an indolizinyl, a pyrazolyl, an oxazolyl, an isoxazolyl, a benzoxazolyl, a thiazolyl, a benzothiazolyl, an isothiazolyl, a pyridazinyl, a pyrimidinyl, a pyrazinyl, a triazinyl, a cinnolinyl, a phtalazinyl, a quinazolinyl, a chromenonyl, a coumarinyl, a quinolonyl, all optionally substituted with one or more substituents selected among hydroxyl, methyl fluoride, methyl chloride, methyl bromide, methyl iodide, ethyl fluoride, ethyl chloride, ethyl bromide, methyl difluoride, methyl dichloride, methyl chlorofluoride, methyl bromochlorofluoride, 2-chloropropyl, fluorocyclopentyl, (dibromomethyl)cyclohexyl, 2-iodo-2-methyl propyl, 2,4-dibromopentyl, methyl trifluoride, methyl trichloride, methyl tribromide, methyl triiodide; methyloxy, ethyloxy, propyloxy, butyloxy, pentyloxy, hexyloxy, octyloxy, isopropyloxy, 2-methyl-1-propyloxy, 2-methyl-2-propyloxy, 2-methyl-1-butyloxy, 3-methyl-1-butyloxy, 2-methyl-3-butyloxy, 2,2-dimethyl-1propyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2,2-dimethyl-1-butyloxy, 3,3-dimethyl-1-butyloxy, 2-ethyl-1-butyloxy, isobutyloxy, t-butyloxy, isopentyloxy, neopentyloxy, benzyloxy, cyclopropyloxy, cyclobutyloxy, cyclopentyloxy, cyclohexyloxy; phenyl, 1-naphtyl, 2-naphtyl; fluorine, chlorine, bromine, iodine; nitro (-NO₂); nitrile (-CN); methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl, optionally substituted with one or more fluorine, chlorine, bromine, iodine; methyloxy, ethyloxy, propyloxy, butyloxy, pentyloxy, hexyloxy, octyloxy, isopropyloxy, 2-methyl-1-propyloxy, 2-methyl-2-propyloxy, 2-methyl-1-butyloxy, 3-methyl-1-butyloxy, 2-methyl-3-butyloxy, 2,2-dimethyl-1propyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2,2-dimethyl-1-butyloxy, 3,3-dimethyl-1-butyloxy, 2-ethyl-1-butyloxy, isobutyloxy, t-butyloxy, isopentyloxy, neopentyloxy, benzyloxy, cyclopropyloxy, cyclobutyloxy, cyclopentyloxy, cyclohexyloxy; methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl; nitro (-NO₂); nitrile (-CN).

In a preferred embodiment of the fifth embodiment, in the carbon labeled organic compound containing a carbon labeled carboxyl group of formula (I) and the organic compound containing a carboxyl group of formula (II), R₁, R₂ and R₃ are, independently, a hydrogen atom; a phenyl substituted with one or more substituents selected among nitro (-NO₂), nitrile (-CN), -CO-phenyl, methyl, ethyl, propyl, methyloxy, ethyloxy, propyloxy, fluorine, chlorine; methyl, ethyl, propyl, methyltrifluoride; benzofuranyl, thiophenyl, benzofuranyl, 2-chromenonyl, 4-chromenonyl, 3-coumarinyl, 4-coumarinyl, 4-quinolonyl, 2-quinolonyl said heteroaryls being optionally substituted by one or more substituents selected among hydroxyl, methoxy, ethoxy, phenyl, fluorine or chlorine atoms, nitrile (-CN), nitro (-NO₂), methyl, ethyl, propyl, methyl trifluoride.

In a **sixth** embodiment of the invention, the process of the invention provides a carbon labeled organic compound containing a carbon labeled carboxyl group of formula (I) and an organic compound containing a carboxyl group of formula (II), wherein R₁ and R₂ form together with the carbon atom to which they are linked a carbonyl (-(C=O)- and R₃ is a hydrogen atom, an aryl having 6 to 14 carbon atoms, a 5 to 10 membered heteroaryl, a C₁-C₈ alkyl, a C₁-C₈ alkyl halide, said aryl, heteroaryl and alkyl groups being optionally substituted.

In this sixth embodiment, in the carbon labeled organic compound containing a carbon labeled carboxyl group of formula (I) and the organic compound containing a carboxyl group of formula (II), R₁ and R₂ form together with the carbon atom to which they are linked a carbonyl (-(C=O)- and R₃ is
- a hydrogen atom,
- a phenyl, 1-naphtyl, 2-naphtyl, dihydronaphtyl, tetrahydronaphtyl, biphenyl, anthryl, phenanthryl, all optionally substituted with one or more substituents selected among hydroxyl, methyl fluoride, methyl chloride, methyl bromide, methyl iodide, ethyl fluoride, ethyl chloride, ethyl bromide, methyl difluoride, methyl dichloride, methyl chlorofluoride, methyl bromochlorofluoride, 2-chloropropyl, fluorocyclopentyl, (dibromomethyl)cyclohexyl, 2-iodo-2-methyl propyl, 2,4-dibromopentyl, methyl trifluoride, methyl trichloride, methyl tribromide, methyl triiodide; fluorine, chlorine, bromine, iodine; a methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1 -butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl; a methyloxy, ethyloxy, propyloxy, butyloxy, pentyloxy, hexyloxy, octyloxy, isopropyloxy, 2-methyl-1-propyloxy, 2-methyl-2-propyloxy, 2-methyl-1-butyloxy, 3-methyl-1-butyloxy, 2-methyl-3-butyloxy, 2,2-dimethyl-1propyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2,2-dimethyl-1-butyloxy, 3,3-dimethyl-1-butyloxy, 2-ethyl-1-butyloxy, isobutyloxy, t-butyloxy, isopentyloxy, neopentyloxy, benzyloxy, cyclopropyloxy, cyclobutyloxy, cyclopentyloxy, cyclohexyloxy; nitro (-NO₂); nitrile (-CN); -CO-phenyl;
- a methyl fluoride, a methyl chloride, a methyl bromide, a methyl iodide, an ethyl fluoride, an ethyl chloride, an ethyl bromide, a methyl difluoride, a methyl dichloride, a methyl chlorofluoride, a methyl bromochlorofluoride, a 2-chloropropyl, a fluorocyclopentyl, a (dibromomethyl)cyclohexyl, a 2-iodo-2-methyl propyl, a 2,4-dibromopentyl, a methyl trifluoride, a methyl trichloride, a methyl tribromide, a methyl triiodide;
- a methyl, an ethyl, a propyl, a butyl, a pentyl, a hexyl, an octyl, an isopropyl, a 2-methyl-1-propyl, a 2-methyl-2-propyl, a 2-methyl-1-butyl, a 3-methyl-1-butyl, a 2-methyl-3-butyl, a 2,2-dimethyl-1-propyl, a 2-methyl-1-pentyl, a 3-methyl-1-pentyl, a 4-methyl-1-pentyl, a 2-methyl-2-pentyl, a 3-methyl-2-pentyl, a 4-methyl-2-pentyl, a 2,2-dimethyl-1-butyl, a 3,3-dimethyl-1-butyl, a 2-ethyl-1-butyl, an isobutyl, a t-butyl, an isopentyl, a neopentyl, a cyclopropyl, a cyclobutyl, a cyclopentyl, a cyclohexyl;
- a furyl, a benzofuranyl, a pyrrolyl, an indolyl, an isoindolyl, an azaindolyl, a thiophenyl, a benzothiophenyl, a 2-pyridyl, a 3-pyridyl, a 4-pyridyl, a 2-quinolinyl, a 1*H*-1,2,3-triazolyl, a 2*H*-1,2,3-triazolyl, a 1*H*-1,2,4-triazolyl, a 4*H*-1,2,4-triazolyl, an isoquinolyl, an imidazolyl, a benzimidazolyl, an indolizinyl, a pyrazolyl, an oxazolyl, an isoxazolyl, a benzoxazolyl, a thiazolyl, a benzothiazolyl, an isothiazolyl, a pyridazinyl, a pyrimidinyl, a pyrazinyl, a triazinyl, a cinnolinyl, a phtalazinyl, a quinazolinyl, a chromenonyl, a coumarinyl, a quinolonyl, all optionally substituted with one or more substituents selected among methyl fluoride, methyl chloride, methyl bromide, methyl iodide, ethyl fluoride, ethyl chloride, ethyl bromide, methyl difluoride, methyl dichloride, methyl chlorofluoride, methyl bromochlorofluoride, 2-chloropropyl, fluorocyclopentyl, (dibromomethyl)cyclohexyl, 2-iodo-2-methyl propyl, 2,4-dibromopentyl, methyl trifluoride, methyl trichloride, methyl tribromide, methyl triiodide; methyloxy, ethyloxy, propyloxy, butyloxy, pentyloxy, hexyloxy, octyloxy, isopropyloxy, 2-methyl-1-propyloxy, 2-methyl-2-propyloxy, 2-methyl-1-butyloxy, 3-methyl-1-butyloxy, 2-methyl-3-butyloxy, 2,2-dimethyl-1propyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2,2-dimethyl-1-butyloxy, 3,3-dimethyl-1-butyloxy, 2-ethyl-1-butyloxy, isobutyloxy, t-butyloxy, isopentyloxy, neopentyloxy, benzyloxy, cyclopropyloxy, cyclobutyloxy, cyclopentyloxy, cyclohexyloxy; phenyl, 1-naphtyl, 2-naphtyl; fluorine, chlorine, bromine, iodine; nitro (-NO₂); nitrile (-CN); methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl, optionally substituted with one or more fluorine, chlorine, bromine, iodine; methyloxy, ethyloxy, propyloxy, butyloxy, pentyloxy, hexyloxy, octyloxy, isopropyloxy, 2-methyl-1-propyloxy, 2-methyl-2-propyloxy, 2-methyl-1-butyloxy, 3-methyl-1-butyloxy, 2-methyl-3-butyloxy, 2,2-dimethyl-1propyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2,2-dimethyl-1-butyloxy, 3,3-dimethyl-1-butyloxy, 2-ethyl-1-butyloxy, isobutyloxy, t-butyloxy, isopentyloxy, neopentyloxy, benzyloxy, cyclopropyloxy, cyclobutyloxy, cyclopentyloxy, cyclohexyloxy; methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl; nitro (-NO₂); nitrile (-CN).

In a preferred embodiment of the sixth embodiment, in the carbon labeled organic compound containing a carbon labeled carboxyl group of formula (I) and the organic compound containing a carboxyl group of formula (II), R₁ and R₂ form together with the carbon atom to which they are linked a carbonyl (-(C=O)- and R₃ is hydrogen atom; a phenyl optionally substituted with one or more substituents selected among hydroxyl, nitrile (-CN), nitro (-NO₂), nitrile (-CN), -CO-phenyl, methyl, ethyl, propyl, methyloxy, ethyloxy, propyloxy, fluorine, chlorine; methyl, ethyl, propyl, methyltrifluoride; benzofuranyl, thiophenyl, benzofuranyl, 2-chromenonyl, 4-chromenonyl, 3-coumarinyl, 4-coumarinyl, 4-quinolonyl, 2-quinolonyl said heteroaryls being optionally substituted with one or more substituents selected among methoxy, ethoxy, phenyl, fluorine or chlorine atoms, nitro (-NO₂), nitrile (-CN), methyl, ethyl, propyl, methyl trifluoride.

In a **seventh** embodiment of the invention, the process of the invention provides a carbon labeled organic compound containing a carbon labeled carboxyl group of formula (I) and an organic compound containing a carboxyl group of formula (II), wherein
R₁ and R₂ form together with the carbon atom to which they are linked an alkene having C₂-C₈ carbon atoms and one or more carbon-carbon double bonds with at least one double bond being alpha to the carboxyl group, said alkene being optionally substituted, and
R₃ is a hydrogen atom, an aryl having 6 to 14 carbon atoms, a 5 to 10 membered heteroaryl, a C₁-C₈ alkyl, a C₁-C₈ alkyl halide, said aryl, heteroaryl and alkyl groups being optionally substituted.

In this seventh embodiment, the process of the invention provides a carbon labeled organic compound containing a carbon labeled carboxyl group of formula (I) and an organic compound containing a carboxyl group of formula (II), wherein
R₁ and R₂ form together with the carbon atom to which they are linked an alkene selected among vinyl, allyl, propenyl, butenyl, pentenyl, hexenyl with at least one double bond being alpha to the carboxyl group, said alkene being optionally substituted with one or more substituents selected among hydroxyl, nitro (-NO₂), nitrile (-CN), methyl, ethyl, propyl, hydroxyl, methyloxy, ethyloxy, propyloxy, fluorine, chlorine, methyltrifluoride, phenyl optionally substituted with one or more substituents selected among fluorine or chlorine atoms, nitro (-NO₂), nitrile (-CN), methyl, ethyl, propyl, methyl trifluoride, and R₃ is
- a hydrogen atom,
- a phenyl, 1-naphtyl, 2-naphtyl, dihydronaphtyl, tetrahydronaphtyl, biphenyl, anthryl, phenanthryl, all optionally substituted with one or more substituents selected among hydroxyl, methyl fluoride, methyl chloride, methyl bromide, methyl iodide, ethyl fluoride, ethyl chloride, ethyl bromide, methyl difluoride, methyl dichloride, methyl chlorofluoride, methyl bromochlorofluoride, 2-chloropropyl, fluorocyclopentyl, (dibromomethyl)cyclohexyl, 2-iodo-2-methyl propyl, 2,4-dibromopentyl, methyl trifluoride, methyl trichloride, methyl tribromide, methyl triiodide; fluorine, chlorine, bromine, iodine; a methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl; a methyloxy, ethyloxy, propyloxy, butyloxy, pentyloxy, hexyloxy, octyloxy, isopropyloxy, 2-methyl-1-propyloxy, 2-methyl-2-propyloxy, 2-methyl-1-butyloxy, 3-methyl-1-butyloxy, 2-methyl-3-butyloxy, 2,2-dimethyl-1propyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2,2-dimethyl-1-butyloxy, 3,3-dimethyl-1-butyloxy, 2-ethyl-1-butyloxy, isobutyloxy, t-butyloxy, isopentyloxy, neopentyloxy, benzyloxy, cyclopropyloxy, cyclobutyloxy, cyclopentyloxy, cyclohexyloxy; nitro (-NO₂); nitrile (-CN); -CO-phenyl;
- a methyl fluoride, a methyl chloride, a methyl bromide, a methyl iodide, an ethyl fluoride, an ethyl chloride, an ethyl bromide, a methyl difluoride, a methyl dichloride, a methyl chlorofluoride, a methyl bromochlorofluoride, a 2-chloropropyl, a fluorocyclopentyl, a (dibromomethyl)cyclohexyl, a 2-iodo-2-methyl propyl, a 2,4-dibromopentyl, a methyl trifluoride, a methyl trichloride, a methyl tribromide, a methyl triiodide;
- a methyl, an ethyl, a propyl, a butyl, a pentyl, a hexyl, an octyl, an isopropyl, a 2-methyl-1-propyl, a 2-methyl-2-propyl, a 2-methyl-1-butyl, a 3-methyl-1-butyl, a 2-methyl-3-butyl, a 2,2-dimethyl-1-propyl, a 2-methyl-1-pentyl, a 3-methyl-1-pentyl, a 4-methyl-1-pentyl, a 2-methyl-2-pentyl, a 3-methyl-2-pentyl, a 4-methyl-2-pentyl, a 2,2-dimethyl-1-butyl, a 3,3-dimethyl-1-butyl, a 2-ethyl-1-butyl, an isobutyl, a t-butyl, an isopentyl, a neopentyl, a cyclopropyl, a cyclobutyl, a cyclopentyl, a cyclohexyl;
- a furyl, a benzofuranyl, a pyrrolyl, an indolyl, an isoindolyl, an azaindolyl, a thiophenyl, a benzothiophenyl, a 2-pyridyl, a 3-pyridyl, a 4-pyridyl, a 2-quinolinyl, a 1*H*-1,2,3-triazolyl, a 2*H*-1,2,3-triazolyl, a 1*H*-1,2,4-triazolyl, a 4*H*-1,2,4-triazolyl, an isoquinolyl, an imidazolyl, a benzimidazolyl, an indolizinyl, a pyrazolyl, an oxazolyl, an isoxazolyl, a benzoxazolyl, a thiazolyl, a benzothiazolyl, an isothiazolyl, a pyridazinyl, a pyrimidinyl, a pyrazinyl, a triazinyl, a cinnolinyl, a phtalazinyl, a quinazolinyl, a chromenonyl, a coumarinyl, a quinolonyl, all optionally substituted with one or more substituents selected among hydroxyl, methyl fluoride, methyl chloride, methyl bromide, methyl iodide, ethyl fluoride, ethyl chloride, ethyl bromide, methyl difluoride, methyl dichloride, methyl chlorofluoride, methyl bromochlorofluoride, 2-chloropropyl, fluorocyclopentyl, (dibromomethyl)cyclohexyl, 2-iodo-2-methyl propyl, 2,4-dibromopentyl, methyl trifluoride, methyl trichloride, methyl tribromide, methyl triiodide; methyloxy, ethyloxy, propyloxy, butyloxy, pentyloxy, hexyloxy, octyloxy, isopropyloxy, 2-methyl-1-propyloxy, 2-methyl-2-propyloxy, 2-methyl-1-butyloxy, 3-methyl-1-butyloxy, 2-methyl-3-butyloxy, 2,2-dimethyl-1propyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2,2-dimethyl-1-butyloxy, 3,3-dimethyl-1-butyloxy, 2-ethyl-1-butyloxy, isobutyloxy, t-butyloxy, isopentyloxy, neopentyloxy, benzyloxy, cyclopropyloxy, cyclobutyloxy, cyclopentyloxy, cyclohexyloxy; phenyl, 1-naphtyl, 2-naphtyl; fluorine, chlorine, bromine, iodine; nitro (-NO₂); nitrile (-CN); methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl, optionally substituted with one or more fluorine, chlorine, bromine, iodine; methyloxy, ethyloxy, propyloxy, butyloxy, pentyloxy, hexyloxy, octyloxy, isopropyloxy, 2-methyl-1-propyloxy, 2-methyl-2-propyloxy, 2-methyl-1-butyloxy, 3-methyl-1-butyloxy, 2-methyl-3-butyloxy, 2,2-dimethyl-1propyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2,2-dimethyl-1-butyloxy, 3,3-dimethyl-1-butyloxy, 2-ethyl-1-butyloxy, isobutyloxy, t-butyloxy, isopentyloxy, neopentyloxy, benzyloxy, cyclopropyloxy, cyclobutyloxy, cyclopentyloxy, cyclohexyloxy; methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl; nitro (-NO₂); nitrile (-CN).

In a preferred embodiment of the seventh embodiment, the process of the invention provides a carbon labeled organic compound containing a carbon labeled carboxyl group of formula (I) and an organic compound containing a carboxyl group of formula (II), wherein
R₁ and R₂ form together with the carbon atom to which they are linked an alkene selected among vinyl, allyl, propenyl, butenyl, with at least one double bond being alpha to the carboxyl group, said alkene being optionally substituted with one or more substituents selected among hydroxyl, nitro (-NO₂), nitrile (-CN), methyl, ethyl, propyl, phenyl optionally substituted with one or more substituents selected among fluorine or chlorine atoms, nitro (-NO₂), nitrile (-CN), methyl, ethyl, propyl, methyl trifluoride, and R₃ is
- a hydrogen atom;
- a methyl, an ethyl, a propyl, a butyl.

In an **eighth** embodiment of the invention, the process of the invention provides a carbon labeled organic compound containing a carbon labeled carboxyl group of formula (I) and an organic compound containing a carboxyl group of formula (II), wherein R₁, R₂ and R₃ form together with the carbon atom to which they are linked an aryl having 6 to 14 carbon atoms, a 5 to 10 membered heteroaryl or a 5 to 10 membered heterocycle, said aryl, heteroaryl and heterocycle groups being optionally substituted.

In this eighth embodiment, in the carbon labeled organic compound containing a carbon labeled carboxyl group of formula (I) and an organic compound containing a carboxyl group of formula (II), R₁, R₂ and R₃ form together with the carbon atom to which they are linked
- a phenyl, 1-naphtyl, 2-naphtyl, dihydronaphtyl, tetrahydronaphtyl, biphenyl, anthryl, phenanthryl, all optionally substituted with one or more substituents selected among hydroxyl, methyl fluoride, methyl chloride, methyl bromide, methyl iodide, ethyl fluoride, ethyl chloride, ethyl bromide, methyl difluoride, methyl dichloride, methyl chlorofluoride, methyl bromochlorofluoride, 2-chloropropyl, fluorocyclopentyl, (dibromomethyl)cyclohexyl, 2-iodo-2-methyl propyl, 2,4-dibromopentyl, methyl trifluoride, methyl trichloride, methyl tribromide, methyl triiodide; fluorine, chlorine, bromine, iodine; a methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl; a methyloxy, ethyloxy, propyloxy, butyloxy, pentyloxy, hexyloxy, octyloxy, isopropyloxy, 2-methyl-1-propyloxy, 2-methyl-2-propyloxy, 2-methyl-1-butyloxy, 3-methyl-1-butyloxy, 2-methyl-3-butyloxy, 2,2-dimethyl-1propyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2,2-dimethyl-1-butyloxy, 3,3-dimethyl-1-butyloxy, 2-ethyl-1-butyloxy, isobutyloxy, t-butyloxy, isopentyloxy, neopentyloxy, benzyloxy, cyclopropyloxy, cyclobutyloxy, cyclopentyloxy, cyclohexyloxy; nitro (-NO₂); nitrile (-CN); -CO-phenyl; sulfonamide (-SO₂-NR₃₅R₃₆) with R₃₅ and R₃₆ being independently an alkyl such as methyl, ethyl, propyl, butyl, pentyl, hexyl;
- furyl, a benzofuranyl, a pyrrolyl, an indolyl, an isoindolyl, an azaindolyl, a thiophenyl, a benzothiophenyl, a 2-pyridyl, a 3-pyridyl, a 4-pyridyl, a 2-quinolinyl, a 1*H*-1,2,3-triazolyl, a 2*H*-1,2,3-triazolyl, a 1*H*-1,2,4-triazolyl, a 4*H*-1,2,4-triazolyl, an isoquinolyl, an imidazolyl, a benzimidazolyl, an indolizinyl, a pyrazolyl, an oxazolyl, an isoxazolyl, a benzoxazolyl, a thiazolyl, a benzothiazolyl, an isothiazolyl, a pyridazinyl, a pyrimidinyl, a pyrazinyl, a triazinyl, a cinnolinyl, a phtalazinyl, a quinazolinyl, a chromenonyl, a coumarinyl, a quinolonyl, all optionally substituted with one or more substituents selected among hydroxyl, methyl fluoride, methyl chloride, methyl bromide, methyl iodide, ethyl fluoride, ethyl chloride, ethyl bromide, methyl difluoride, methyl dichloride, methyl chlorofluoride, methyl bromochlorofluoride, 2-chloropropyl, fluorocyclopentyl, (dibromomethyl)cyclohexyl, 2-iodo-2-methyl propyl, 2,4-dibromopentyl, methyl trifluoride, methyl trichloride, methyl tribromide, methyl triiodide; methyloxy, ethyloxy, propyloxy, butyloxy, pentyloxy, hexyloxy, octyloxy, isopropyloxy, 2-methyl-1-propyloxy, 2-methyl-2-propyloxy, 2-methyl-1-butyloxy, 3-methyl-1-butyloxy, 2-methyl-3-butyloxy, 2,2-dimethyl-1propyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2,2-dimethyl-1-butyloxy, 3,3-dimethyl-1-butyloxy, 2-ethyl-1-butyloxy, isobutyloxy, t-butyloxy, isopentyloxy, neopentyloxy, benzyloxy, cyclopropyloxy, cyclobutyloxy, cyclopentyloxy, cyclohexyloxy; phenyl, 1-naphtyl, 2-naphtyl; fluorine, chlorine, bromine, iodine; nitro (-NO₂); nitrile (-CN); methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl, optionally substituted with one or more fluorine, chlorine, bromine, iodine; methyloxy, ethyloxy, propyloxy, butyloxy, pentyloxy, hexyloxy, octyloxy, isopropyloxy, 2-methyl-1-propyloxy, 2-methyl-2-propyloxy, 2-methyl-1-butyloxy, 3-methyl-1-butyloxy, 2-methyl-3-butyloxy, 2,2-dimethyl-1propyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2,2-dimethyl-1-butyloxy, 3,3-dimethyl-1-butyloxy, 2-ethyl-1-butyloxy, isobutyloxy, t-butyloxy, isopentyloxy, neopentyloxy, benzyloxy, cyclopropyloxy, cyclobutyloxy, cyclopentyloxy, cyclohexyloxy; methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl; nitro (-NO₂); nitrile (-CN).
- morpholinyl, piperidinyl, piperazinyl, pyrrolidinyl, imidazolidinyl, imidazolinyl, pyrazolidinyl, tetrahydrofuranyl, tetrahydropyranyl, thianyl, oxazolidinyl, isoxazolidinyl, thiazolidinyl and isothiazolidinyl, phtalimidyl, indolinyl, all being optionally substituted by one or more substituents selected among hydroxyl, methyl fluoride, methyl chloride, methyl bromide, methyl iodide, ethyl fluoride, ethyl chloride, ethyl bromide, methyl difluoride, methyl dichloride, methyl chlorofluoride, methyl bromochlorofluoride, 2-chloropropyl, fluorocyclopentyl, (dibromomethyl)cyclohexyl, 2-iodo-2-methyl propyl, 2,4-dibromopentyl, methyl trifluoride, methyl trichloride, methyl tribromide, methyl triiodide; fluorine, chlorine, bromine, iodine; a methyl, ethyl, propyl, butyl, pentyl, hexyl, octyl, isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl, benzyl, cyclopropyl, cyclobutyl, cyclopentyl, cyclohexyl; a methyloxy, ethyloxy, propyloxy, butyloxy, pentyloxy, hexyloxy, octyloxy, isopropyloxy, 2-methyl-1-propyloxy, 2-methyl-2-propyloxy, 2-methyl-1-butyloxy, 3-methyl-1-butyloxy, 2-methyl-3-butyloxy, 2,2-dimethyl-1propyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2,2-dimethyl-1-butyloxy, 3,3-dimethyl-1-butyloxy, 2-ethyl-1-butyloxy, isobutyloxy, t-butyloxy, isopentyloxy, neopentyloxy, benzyloxy, cyclopropyloxy, cyclobutyloxy, cyclopentyloxy, cyclohexyloxy; nitro (-NO₂); nitrile (-CN); a phenyl, 1-naphtyl, 2-naphtyl.

In a preferred embodiment of the eighth embodiment, in the carbon labeled organic compound containing a carbon labeled carboxyl group of formula (I) and an organic compound containing a carboxyl group of formula (II), R₁, R₂ and R₃ form together with the carbon atom to which they are linked
- a phenyl, 1-naphtyl, 2-naphtyl, all optionally substituted with one or more substituents selected among hydroxyl; methyl trifluoride, methyl trichloride; fluorine, chlorine; a methyl, ethyl, propyl, butyl, isopropyl, isobutyl, t-butyl; a methyloxy, ethyloxy, propyloxy; nitro (-NO₂); nitrile (-CN); -CO-phenyl; sulfonamide (-SO₂-NR₃₅R₃₆) with R₃₅ and R₃₆ being independently an alkyl such as methyl, ethyl, propyl, butyl, pentyl, hexyl;
- a furanyl, a benzofuranyl, a pyrrolyl, an indolyl, a thiophenyl, a benzothiophenyl, all optionally substituted with one or more substituents selected among hydroxyl; methyl trifluoride, methyl trichloride; fluorine, chlorine; a methyl, ethyl, propyl, butyl, isopropyl, isobutyl, t-butyl; a methyloxy, ethyloxy, propyloxy; nitro (-NO₂); nitrile (-CN).

The process of the invention provides a carbon labeled organic compound containing a carbon labeled carboxyl group of formula (I) and an organic compound containing a carboxyl group of formula (II), wherein M₁ is a hydrogen atom, a silver cation (Ag⁺), an alkaline cation selected from lithium (Li⁺), sodium (Na⁺), potassium (K⁺), rubidium (Rb⁺), or cesium (Cs⁺). In particular, M₁ is a hydrogen atom, an alkaline cation selected sodium (Na⁺), potassium (K⁺), or cesium (Cs⁺).

In a preferred embodiment of the invention, the organic compound containing a carboxyl group of formula (II), used for the synthesis of a carbon labeled organic compound containing a carbon labeled carboxyl group according to formula (I), is 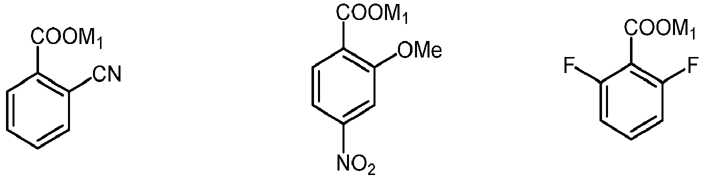 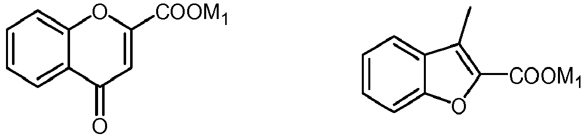 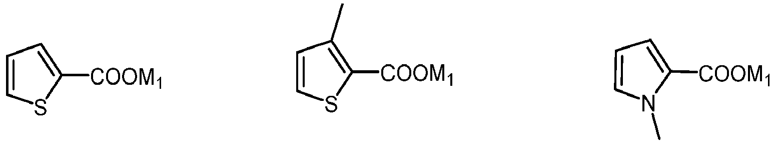 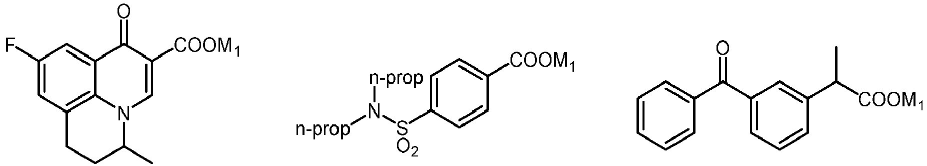 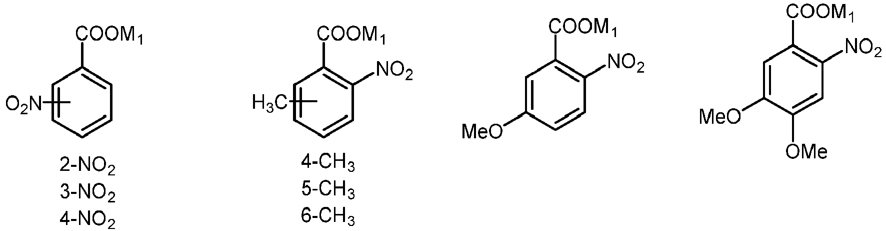 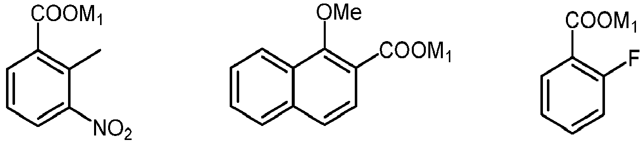 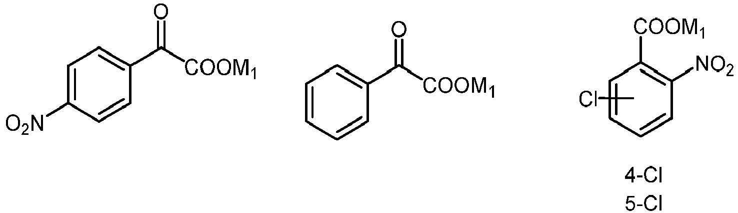 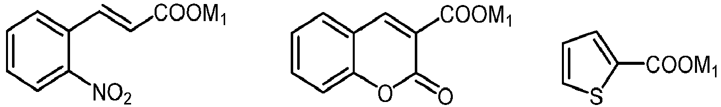 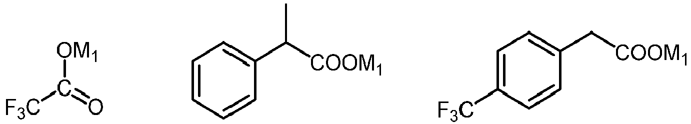 wherein M₁ is a hydrogen atom, a silver cation (Ag⁺), an alkaline cation selected from lithium (Li⁺), sodium (Na⁺), potassium (K⁺), rubidium (Rb⁺), or cesium (Cs⁺), in particular, M₁ is a hydrogen atom, an alkaline cation selected sodium (Na⁺), potassium (K⁺), or cesium (Cs⁺).

The process of the invention disposes of several variables which can be changed independently or together to maximize the reaction outcome: the nature of the catalyst system, the nature of the organic compounds containing a carboxyl group of formula (II), the *CO₂ source, the concentration of the reactants involved and the temperature. The variations can be combined, meaning that the catalyst system can be changed at the same time as the organic compound containing a carboxyl group of formula (II) or the *CO₂ source. Pressure, temperature and solvent can also be declined independently of the nature of the catalyst system, organic compound containing a carboxyl group or the *CO₂ source.

In an embodiment of the present invention, the source of *CO₂ is a gas. In this embodiment, the *CO₂ pressure in the reaction vessel is between 0.5 to 100 bar (50 kPa to 10 MPa), in particular, between 1 and 20 bar.

In an embodiment, the *CO₂ source is a *CO₂ surrogate resulting from the acidification of a carbonate selected among Ba*CO₃, NaH*CO₃, Na₂*C₂O₅, K₂*C₂O₅, Na₂*CO₃ and K₂*CO₃ with an acid selected among HCl, H₂SO₄, HNO₃. The amount of acid added is in general in excess, in particular, between 5 and 100 mole protons/mole carbon-atoms. The molar ratio of the carbonate used in the acidification reaction and the organic compound containing a carboxyl group of formula (II) is between 0.25 and 50, in particular, between 0.5 and 5. (Chapter 5 of Preparation of Compounds Labeled with Tritium and Carbon-14, Rolf Voges, J. Richard Heys and Thomas Moenius © 2009 John Wiley & Sons).

The organic compounds containing a carboxyl group of formula (II), the inorganic salt of formula (III) and the ligands of formula (IV) to (V) of the catalyst system are in general easily synthesized or commercially available.

In all of the embodiments of the invention, the catalyst system is present in an amount of 1 to 100 mole percent (mol %), in particular, between 5 and 25 mole percent (mol %), with respect to compound (II).

In all of the embodiments of the invention, in the catalyst system, the molar ratio of the inorganic salt of formula (III) and the ligands of formula (IV) to (V) is between 1 to 100, in particular, between 1 and 20, more particularly, between 1 and 5.

The process of the invention can occur in a solvent or a mixture of at least two solvents selected among diethylether, dimethylether, dioxane, N-methyl-pyrolidone (NMP), benzene, ethylacetate, chloroform, acetone, nitromethane, dimethylformamide (DMF), dimethylsulfoxide (DMSO), N,N-dimethylacetamide (DMA) acetonitrile, tetrahydrofurane (THF), dichloromethane (DCM), or toluene.

The reaction temperature can be between 50 and 200°C, preferably between 50 and 150°C.

The reaction time can be between 5 minutes and 48 hours, preferably between 5 minutes to 24 hours.

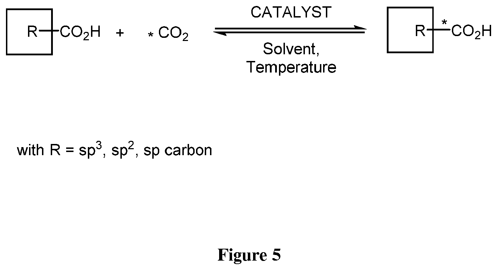

The process of the invention takes places preferably, under an atmosphere of labeled CO₂ and in the presence of a catalyst system. This process has the advantage of being applicable to all organic compounds containing a carboxylic acid functionality, independently of the orbital hybridization of the carbon atom attached to the carboxyl function (sp, sp² or sp³ carbon). This is illustrated in Figure 5.

Where necessary, the carbon labeled organic compound containing a carbon labeled carboxyl group of formula (I) obtained by the process of the invention may be purified. Purification of the desired compound may be achieved by conventional methods such as extraction from the aqueous quench and subsequent column chromatography, or by distillation or recrystallization depending on the nature of the carbon labeled organic compound containing a carbon labeled carboxyl group of formula (I). The skilled person is able to choose the adapted purification method taking into account the nature of the carboxylic group.

All of the combinations of the embodiments disclosed are encompassed by the present invention.

Isotopically carbon labeled organic compounds containing a carbon labeled carboxyl group according to formula (I) represent a special interest in several domains as for example, in life science (elucidation and study of enzymatic mechanism, biosynthetic mechanisms, biochemistry, etc.), in environmental science (waste tracing), chemical research (study and elucidation of reaction mechanism) or further the research and development of new pharmaceutics and therapeutics.

In the context of the present invention, isotopes are two atoms of the same element, which differ in number of neutrons, but which have the same number of protons and electrons. Therefore the chemical properties of isotopes of the same element are almost the same. However slight differences in reaction kinetics can exist, when one atom of a reagent is changed for one of its isotopes. As the nucleus of isotopes does not possess of the same number of neutrons, the mass of atoms changes, which might lead to radioactivity and those isotopes are therefore noted as radioisotopes. In the context of this invention the term isotope can include radioisotopes and vice versa.

Radiolabeling consists of adding an isotope to a molecule or compound, which allows to follow its evolution and/or fixation of the labeled molecules, for example in an organ. The radiotracer(s) is/are the radioactive element(s) in a molecule, which allow(s) to follow the pathway of this substance for example in an organ. The process of the invention can therefore give access to carbon labeled organic compounds containing a carbon labeled carboxyl group according to formula (I) incorporating a ¹¹C, ¹³C or ¹⁴C. The use of labeled molecules is detailed in the literature (Pleiss, R. Voges, "Synthesis and Applications of Isotopically Labeled Compounds, Volume 7". Wiley-VCH, 2001 ; R. Voges, J. R. Heys, T. Moenius, "Preparation of Compounds Labeled with Tritium and Carbon-14". Wiley-VCH: Chippenham (UK), 2009).

Another aspect of the invention concerns the use of carbon labeled organic compounds containing a carbon labeled carboxyl group according to formula (I) obtained by a process according to the invention in the manufacture of pharmaceuticals and agrochemicals, in particular pharmaceuticals and agrochemicals having a free carboxylic acid functionality.

Another aspect of the invention relates to a process for manufacturing labeled pharmaceuticals and agrochemicals, in particular pharmaceuticals and agrochemicals having a free carboxylic acid functionality, comprising a step of synthesis of carbon labeled organic compounds containing a carbon labeled carboxyl group according to formula (I) obtained by a process according to the invention. This manufacturing process may optionally comprise a step of solvent extraction and/or purification.

A still another aspect of the invention concerns further relates to a process for producing tracers, characterized in that it comprises a step of synthesis of carbon labeled organic compounds containing a carbon labeled carboxyl group according to formula (I) obtained by the process according to the invention. This production process may optionally comprise a step of solvent extraction and/or purification.

Other features and advantages of the present invention appear from the figures and the following non limiting examples.
Figure 1 represents the labeling of Valsartan (angiotensin II receptor antagonist) with ³H in one single operation using the appropriate catalyst and readily commercially available Valsartan.
Figure 2 represents the labeling of Valsartan (angiotensin II receptor antagonist) with ¹⁴C in eight steps using the appropriate catalyst and readily commercially available Valsartan.
Figure 3 represents the mechanism of the catalytic dynamic carbon isotope exchange with CO₂ according to the process of the invention.
Figure 4 represents the principle for the dynamic carbon isotope exchange according to the invention.

### EXAMPLES

NMR data were recorded with RMN Bruker Avance 400 Mhz with topspin 2.1.

Mass data were recorded with a Waters ZQ 2000, with electrospray positif/negatif source. Direct I ntroduction (4 min), ACN/MeOH 50/50 + 1/1000 HCO₂H.

Glove box model: mb-unilab plus sp (http://www.mbraun.com/products/ glovebox-workstations/unilab-glovebox).

Wilmad Young NMR tube were purchased from Sigma Aldrich (https://www.sigmaaldrich.com/catalog/product/aldrich/z514160?lang=fr&region=FR)

All carboxylic acids substrates, ligands and catalysts are commercially available and purchased from different Sigma-Aldrich, Alfa Aesar and Acros Organics.

¹³CO₂ is commercially available from Sigma Aldrich. It was charger to the TRITEC cartridge according to the specifications of the manifold (see http://www.rctritec.com/en/tritium-handling-technology/c-14-manifold-system.html)

For the preparation of ¹¹CO₂, see Clin Transl Imaging, 2017, 5, 275-289.

¹⁴CO₂ was purchased from TRITEC, for the preparation of ¹⁴CO₂ and BaCO₂ see: Preparation of Compounds Labeled with Tritium and Carbon-14 Rolf Voges, J. Richard Heys and Thomas Moenius© 2009 John Wiley & Sons, page 211 (chapter 5).

### General protocol

### 1. Cesium salt preparation

Adapted from the literature (Gérard Cahiez, Alban Moyeux, Olivier Gager, Mal Poizatb, Adv. Synth. Catal. 2013, 355, 790 - 796): a flask was charged with the desired organic compound containing a carboxyl group (2- nitrobenzoic acid) and methanol (5-10 mL). After stirring for 5 min, Cs₂CO₃ (1eq.) was slowly added to the solution. The reaction mixture was then stirred for 1 h at room temperature (20 + 5°C). Methanol was removed under vacuum and the resulting solid was dried in a vacuum oven at 40 °C for 24h to provide cesium 2-nitrobenzoate.

The dryness of the resulting cesium salt was checked: an argon-filled glovebox, a Wilmad® NMR tube (5 mm diam. 7 In.) with Young valve was charged with the cesium salt and a proton NMR was performed adding internal standard (trimethoxybenzene, 3 eq.) in MeOD. The peaks integrations revealed the salt purity, that was useful to calculate the correct amount of catalyst loading.

### 2. Potassium salt preparation

In an argon-filled glovebox, a Schlenk flask was charged with the desired organic compound containing a carboxyl group (2- nitrobenzoic acid) and THF (5-10 mL). After stirring for 5 min, KH (1eq.) was slowly added to the solution. The reaction mixture was then stirred for 1-2 h at room temperature. THF was removed under vacuum and the resulting solid was dried under vacuum for 24h to provide potassium 2-nitrobenzoate.

The dryness of the resulting potassium salt was checked: an argon-filled glovebox, a Wilmad® NMR tube (5 mm diam. 7 In.) with Young valve was charged with potassium 2-nitrobenzoate and a proton NMR was performed adding internal standard (trimethoxybenzene, 3 eq.) in TDF. The peaks integrations revealed the salt purity, that was useful to calculate the correct amount of catalyst loading.

### 3. General Carbon Isotopic Exchange Reaction

An argon-filled glovebox, a Wilmad® NMR tube (5 mm diam. 7 In.) with Young valve, was charged with the cesium or potassim salt of desired organic compound containing a carboxyl group (1eq), the ligand (E)-2-(4-phenyl-4,5-dihydrooxazol-2-yl)-2-(4-phenyloxazolidin-2-ylidene)acetonitrile (6.6mg, 2x10⁻⁵mol, 20%mol) and copper bromide (I) (2.9 mg, 2x10⁻⁵mol, 20%mol). Subsequently, dry DMSO (0.5ml) is added to the mixture.

The loaded NMR tube was removed from the glovebox and connected to carbon TRITEC manifold according to the disclosure in:

### http://www.rctritec.com/en/tritium-handling-technology/c-14-manifold-system.html

The NMR tube mixture was frozen using a liquid nitrogen bath and the system is degassed under high vacuum. The NMR tube is then charged with labeled CO₂ (3eq.). The Wilmad® NMR tube is subsequently sealed and the reaction mixture was allowed to warm at room temperature 20±5°C) (2 min) and stirred at 150 °C for 2 hours.

The reaction mixture is then allowed to cool at room temperature (20±5°C) and quenched with HCl 1M (5 mL) and stirred for 2 minutes. Then the mixture was extracted with ethyl acetate (3-5 mL, 3 times). The combined organic layers were extracted with sodium hydroxide (1M) or with a saturated solution of sodium bicarbonate, until basic pH was reached. The basic aqueous phase was slowly acidified to pH 2 with HCl (2M) and extracted with ethyl acetate (3-5 mL, 3 times).

The combined organic layers in ethyl acetate were dried (MgSO₄) and concentrated under reduced pressure to obtain the isotopically enriched final compound.

### EXAMPLE 1: Synthesis of ¹³C-2-Nitrobenzoic acid

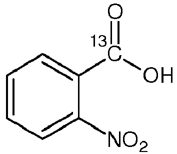

The title compound was synthesized according to general protocol from the cesium salt of 2-nitrobenzoic acid (29.9mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described. After the reaction and purification the enriched final compound with 50% yield (8.3mg) and 72% of isotopic enrichment.
**¹H NMR** (400MHz, DMSO) δ 7.98 (d, *J*=7.8 Hz, 1H), 7.88-7.84 (m, 1H), 7.82-7.75 (m, 1H)
**¹³C NMR** (400 MHz, DMSO) δ 165.9, 148.4, 133.1, 132.4, 129.9, 127.2 (*J*=73.4 Hz), 123.7
**Mass** M-1= 166, [M-1]+1 (13C): 72.1%

### EXAMPLE 2: Synthesis of ¹³C-4-Nitrobenzoic acid

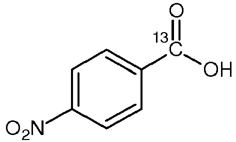

The title compound was synthesized according to general protocol from the cesium salt of 4-nitrobenzoic acid (29.9mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described. After the reaction and purification the enriched final compound with 65% yield (10.8mg) and 14% of isotopic enrichment.
**1H NMR** (400MHz, DMSO) δ 8.33 (dt, *J*=9.0 Hz, 1.8 Hz, 2H), 8.1 (dt, *J*=8.7 Hz, 2.2 Hz, 2H),
**13C NMR** (400 MHz, DMSO) δ 165.8, 150.1, 136.4, 130.7, 123.8,
**Mass** M-1= 166, [M-1]+1 (13C): 14%

### EXAMPLE 3: Synthesis of ¹³C-6-Methyl-2-Nitrobenzoic acid

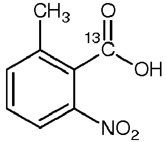

The title compound was synthesized according to general protocol from the cesium salt of 6-Methyl-2-Nitrobenzoic acid (31.3mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described. After the reaction and purification the enriched final compound with 48% yield (8.7mg) and 73% of isotopic enrichment.
**1H NMR** (400MHz, DMSO) δ 7.9 (d, *J*=8.2 Hz, 1H), 7.7 (d, *J*=7.6 Hz, 1H), 7.5 (t, J=7.9 Hz, 1H), 2.37 (s, 3H)
**13C NMR** (400 MHz, DMSO) δ 167.3, 145.9, 136.7, 136.4, 129.8, 121.8, 19.0
**Mass** M-1= 180, [M-1]+1 (13C): 73%

### EXAMPLE 4: Synthesis of ¹³C-5-Methyl-2-Nitrobenzoic acid

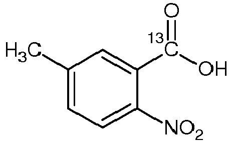

The title compound was synthesized according to general protocol from the cesium salt of 5-Methyl-2-Nitrobenzoic acid (31.3mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described. After the reaction and purification the enriched final compound with 45% yield (8.3mg) and 44% of isotopic enrichment.
**1H NMR** (400MHz, DMSO) δ 7.9 (d, *J*=8.3 Hz, 1H), 7.6 (d, *J*=2.3 Hz, 1H), 7.5 (dd, *J*=8.4 Hz, 1.3 Hz, 1H)
**13C NMR** (400 MHz, DMSO) δ 166.3, 145.6, 144.3, 132.1, 129.8, 128.1 (*J*=73.3 Hz), 123.8, 20.7
**Mass** M-1= 180, [M-1]+1 (13C): 44%

### EXAMPLE 5: Synthesis of ¹³C-4-Methyl-2-Nitrobenzoic acid

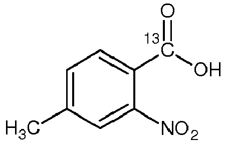

The title compound was synthesized according to general protocol from the cesium salt of 4-Methyl-2-Nitrobenzoic acid (31.3mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described. After the reaction and purification the enriched final compound with 42% yield (7.5 mg) and 57% of isotopic enrichment.
**1H NMR** (400MHz, DMSO) δ 7.8 (d, *J*=2.3 Hz, 1H), 7.7 (s, 1H), 7.6 (d, *J*=7.9 Hz, 1H), 2.43 (s, 3H)
**13C NMR** (400 MHz, DMSO) δ 165.4, 148.7, 143.3, 133.9, 129.8, 123.6, 123.5(d, *J*=9.9 Hz), 20.4
**Mass** M-1= 180, [M-1]+1 (13C): 57%

### EXAMPLE 6: Synthesis of ¹³C-4-Methoxy-2-Nitrobenzoic acid

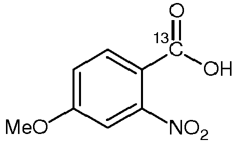

The title compound was synthesized according to general protocol from the cesium salt of 5-Methoxy-2-Nitrobenzoic acid (32.9mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described. After the reaction and purification the enriched final compound with 53% yield (10.3 mg) and 39% of isotopic enrichment.
**1H NMR** (400MHz, DMSO) δ 8.0 (d, *J*=8.5 Hz, 1H), 7.2-7.1 (m, 2H), 3.9 (s, 3H),
**13C NMR** (400 MHz, DMSO) δ 166.6, 163.1, 139.5, 132 (*J*=38.1 Hz), 126.6, 115.9, 113.9, 56.5
**Mass** M-1= 166, [M-1]+1 (13C): 46%

### EXAMPLE 7: Synthesis of ¹³C-4,5-dimethoxy-2-Nitrobenzoic acid

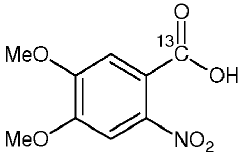

The title compound was synthesized according to general protocol from the cesium salt of 4,5-dimethoxy-2-Nitrobenzoic acid (35.9mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described. After the reaction and purification the enriched final compound with 41% yield (9.4mg) and 10% of isotopic enrichment.
**1H NMR** (400MHz, DMSO) δ 8.0 (d, *J*=8.5 Hz, 1H), 7.2-7.1 (m, 2H), 3.9 (s, 3H),
**13C NMR** (400 MHz, DMSO) δ 166.6, 163.1, 139.5, 132 (*J*=38.1 Hz), 126.6, 115.9, 113.9, 56.5
**Mass** M-1= 166, [M-1]+1 (13C): 10%

### EXAMPLE 8: Synthesis of ¹³C-4-chloro-2-Nitrobenzoic acid

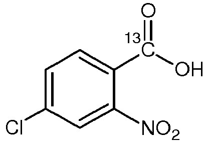

The title compound was synthesized according to general protocol from the cesium salt of 4-chloro-2-Nitrobenzoic acid (33.3mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described. After the reaction and purification the enriched final compound with 29% yield (5.6mg) and 12.5% of isotopic enrichment.
**1H NMR** (400MHz, DMSO) δ 8.1 (d, *J*=1.6 Hz, 1H), 7.9-7.8 (m, 2H),
**13C NMR** (400 MHz, DMSO) δ 164.5, 150.7, 136.5, 132.5, 131.6, 124.3, 123.5 **Mass** M-1= 166, [M-1]+1 (13C): 12.5%

### EXAMPLE 9: Synthesis of ¹³C-3-Nitrobenzoic acid

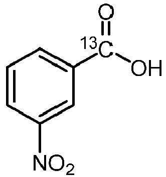

The title compound was synthesized according to general protocol from the cesium salt of 3-nitrobenzoic acid (29.9mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described, the temperature was set to 190°C. After the reaction and purification the enriched final compound with 88% yield (14.6mg) and 20% of isotopic enrichment.
**1H NMR** (400MHz, DMSO) δ 13.6 (broad peak, 1H), 8.61 (s, 1H), 8.47 (dd, *J*=8.0, 1.7 Hz, 1H), 8.35 (d, *J*=7.7 Hz, 1H), 7.8 (t, *J*=8.0, 7.8 Hz, 1H)
**13C NMR** (400 MHz, DMSO) δ 165.9, 147.8, 133.1, 135.4, 132.5, 127.4, 123.7
**Mass** M-1= 166, [M-1]+1 (13C): 20%

### EXAMPLE 10: Synthesis of ¹³C-3-methylbenzofuran-2-carboxylic acid

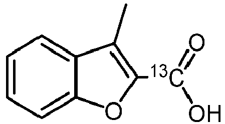

The title compound was synthesized according to general protocol from the cesium salt of 3-methylbenzofuran-2-carboxylic acid (30.8mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵ mol) and copper bromide (2.9mg, 2x10⁻⁵mol) after that dry DMSO 0.5ml is added to the mixture.

Hence loaded with ¹³CO₂ as described. After the reaction and purification the enriched final compound with 69% yield (12.1mg) and 37% of isotopic enrichment.
**1H NMR** (400MHz, DMSO) δ 13.4 (broad peak, 0.7H), 7.7 (d, *J*=7.8 Hz, 1H), 7.6 (d, *J*=8.3 Hz, 1H), 7.5 (t, *J*=7.8 Hz, 1H), 7.3 (t, *J*=7.8 Hz, 1H), 2.5 (s, 3H)
**13C NMR** (400 MHz, DMSO) δ 161.4, 153.4, 128.8, 127.5, 123.7, 123.1, 121.3, 111.8, 10
**Mass** M-1= 175.16, [M-1]+1 (13C): 37%

### EXAMPLE 11: Synthesis of ¹³C-thiophene-2-carboxylic acid

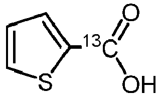

The title compound was synthesized according to general protocol from the cesium salt of thiophene-2-carboxylic acid (25.9mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described. After the reaction and purification the enriched final compound with 30% yield (3.8mg) and 30% of isotopic enrichment.
**1H NMR** (400MHz, DMSO) δ 13.1 (broad peak, 0.9H), 7.8 (d, *J*=4.7 Hz, 1H), 7.7 (d, *J*=3.4 Hz, 1H), 7.2 (t, *J*=4.2 Hz, 1H)
**13C NMR** (400 MHz, DMSO) δ 162.9, 134.9, 133.1, 133.0, 128.4
**Mass** M-1= 127.14, [M-1]+1 (13C): 30%

### EXAMPLE 12: Synthesis of ¹³C-3-methylthiophene-2-carboxylic acid

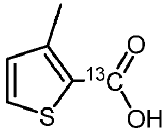

The title compound was synthesized according to general protocol from the cesium salt of 3-methylthiophene -2-carboxylic acid (27.3mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described. After the reaction and purification the enriched final compound with 36% yield (5.1mg) and 17% of isotopic enrichment.
**1H NMR** (400MHz, DMSO) δ 12.8 (broad peak, 1H), 7.7 (d, *J*=5.5 Hz, 1H), 7.0 (d, *J*=4.4 Hz, 1H), 2.4 (s, 3H)
**13C NMR** (400 MHz, DMSO) δ 163.7, 145.0, 132.1, 130.8, 127.4, 15.6
**Mass** M-1= 141.16, [M-1]+1 (13C): 17%

### EXAMPLE 13: Synthesis of ¹³C-2-cyanobenzoic acid

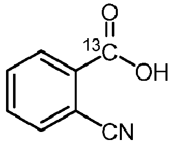

The title compound was synthesized according to general protocol from the cesium salt of 2-cyanobenzoic acid (27.8mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described. After the reaction and purification the enriched final compound with 63% yield (9.3mg) and 10% of isotopic enrichment.
**1H NMR** (400MHz, DMSO) δ 13.8 (broad peak, 1H), 8.1 (d, *J*=7.8 Hz, 1H), 7.9 (d, *J*=7.9 Hz, 1H), 7.8 (m, 2H)
**13C NMR** (400 MHz, DMSO) δ 165.2, 145.0, 135.0, 133.2, 133.0, 130.9, 130.7, 117.7, 111.6
**Mass** M-1= 146, [M-1]+1 (13C): 10%

### EXAMPLE 14: Synthesis of ¹³C-1-methoxy-2-naphthoic acid

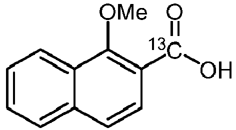

The title compound was synthesized according to general protocol from the cesium salt of 1-methoxy-2-naphthoic acid (33.3mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described. After the reaction and purification the enriched final compound with 50% yield (10.1mg) and 25% of isotopic enrichment.
**1H NMR** (400MHz, DMSO) δ 13.0 (broad peak, 0.9H), 8.2 (dd, *J*=7.2, 2.0, Hz, 1H), 7.9 (dd, *J*=6.8, 2.0 Hz, 1H), 7.7 (dd, *J*=20.0, 8.6 Hz, 2H) 7.6 (m, 2H)
**13C NMR** (400 MHz, DMSO) δ 160.2, 149.6, 128.8, 120.2, 119.9, 119.5, 118.3, 118.2, 115.2, 114.8, 111.4, 54.21
**Mass** M-1= 201.2, [M-1]+1 (13C): 25%

### EXAMPLE 15: Synthesis of ¹³C-2-oxo-2H-chromene-3-carboxylic acid

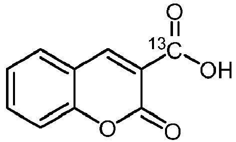

The title compound was synthesized according to general protocol from the cesium salt of 2-oxo-2H-chromene-3-carboxylic acid (32.1mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described. After the reaction and purification the enriched final compound with 56% yield (10.6mg) and 40% of isotopic enrichment.
**1H NMR** (400MHz, DMSO) δ 13.3 (broad peak, 0.8H), 8.7 (s, 1H), 7.9 (dd, *J*=7.8, 1.5, Hz, 1H), 7.7 (td, *J*=7.9, 2.0 Hz, 1H), 7.44 (d, J= 8.5 Hz, 1H) 7.4 (dd, *J*=7.4, 1.1, Hz, 1H)
**13C NMR** (400 MHz, DMSO) δ 164.0, 156.6, 154.4, 148.4, 134.3, 130.1, 124.8, 118.3, 117.9, 116.1
**Mass** M-1= 189.15, [M-1]+1 (13C): 40%

### EXAMPLE 16: Synthesis of ¹³C-1-methyl-1H-pyrrole-2-carboxylic acid

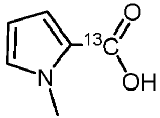

The title compound was synthesized according to general protocol from the cesium salt of 1-methyl-1H-pyrrole-2-carboxylic acid (25.6mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described. After the reaction and purification the enriched final compound with 42% yield (5.3mg) and 16% of isotopic enrichment.
**1H NMR** (400MHz, DMSO) δ 12.1 (broad peak, 0.8H), 7.0 (t, *J*=2.1 Hz, 1H), 6.7 (dd, *J*=4.0, 1.8, Hz, 1H), 6.0 (dd, *J*=4.2, 2.4, Hz, 1H), 3.88 (s, 3H)
**13C NMR** (400 MHz, DMSO) δ 162.0, 129.7, 122.4, 117.3, 107.1, 36.3
**Mass** M-1= 124.13, [M-1]+1 (13C): 16%

### EXAMPLE 17 : Synthesis of ¹³C-2-methyl-3-nitrobenzoic acid

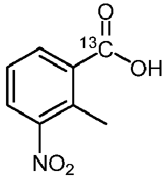

The title compound was synthesized according to general protocol from the cesium salt of 2-methyl-3-nitrobenzoic acid (31.2mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry NMP/DMSO (8:2 mixture) 0.5ml is added to the mixture.

Hence loaded with ¹³CO₂ as described. After the reaction and purification the enriched final compound with 50% yield (9mg) and 48% of isotopic enrichment.
**1H NMR** (400MHz, DMSO) δ 13.5 (broad peak, 0.8H), 8.0 (d, *J*=8.5 Hz, 2H), 7.5 (t, *J*=7.8 Hz, 1H), 2.5 (s, 3H)
**13C NMR** (400 MHz, DMSO) δ 167.8, 151.5, 134.4 (t, *J*=37 Hz) 133.2, 130.8, 127.1, 126.2, 15.5
**Mass** M-1= 180.15, [M-1]+1 (13C): 48%

### EXAMPLE 18: Synthesis of ¹³C-2,6-difluorobenzoic acid

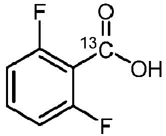

The title compound was synthesized according to general protocol from the cesium salt of 2,6-difluorobenzoic acid (28.9mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described, reaction time 1h. After the reaction and purification the enriched final compound with 25% yield (3.95mg) and 40% of isotopic enrichment.
**1H NMR** (400MHz, DMSO) δ 13.9 (broad peak, 0.9H), 7.5 (m, 1H), 7.2 (t, *J*=7.6 Hz, 1H)
**13C NMR** (400 MHz, DMSO) δ 165, 162.1, 132.9, 112.4, 112.1
**Mass** M-1= 157, [M-1]+1 (13C): 40%

### EXAMPLE 19: Synthesis of ¹³C-2-methoxy-4-nitrobenzoic acid

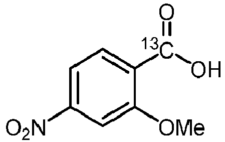

The title compound was synthetized according to general protocol from the cesium salt of 2-methoxy-4-nitrobenzoic acid (32.8mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry NMP/DMSO (8:2 mixture) 0.5ml is added to the mixture.

Hence loaded with ¹³CO₂ as described. After the reaction and purification the enriched final compound with 81% yield (15.5mg) and 15% of isotopic enrichment.
**1H NMR** (400MHz, DMSO) δ 13.4 (broad peak, 0.9H), 7.87-7.799 (m, 3H), 3.9 (s, 3H)
**13C NMR** (400 MHz, DMSO) δ 166.3, 157.1, 149.6, 130.8, 128.0, 115.1, 106.8, 56.56
**Mass** M-1= 196, [M-1]+1 (13C): 15%

### EXAMPLE 20: Synthesis of ¹³C-2-(4-(trifluoromethyl)phenyl)acetic acid

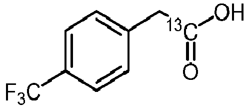

The title compound was synthesized according to general protocol from the cesium salt of 2-(4-(trifluoromethyl)phenyl)acetic acid (32.8mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described, reaction temperature 100 °C. After the reaction and purification the enriched final compound with 22% yield (4.5mg) and 56% of isotopic enrichment.
**1H NMR** (400MHz, DMSO) δ 7.6 (d, *J*=7.4 Hz, 2H), 7.5 (d, *J*=7.4 Hz, 2H), 3.7 (t, *J*=3.9 Hz, 2H)
**13C NMR** (400 MHz, DMSO) δ 172.2, 140.0, 130.3, 127.0, 124.5, 123.0
**Mass** M-1= 203.15, [M-1]+1 (13C): 56%

### EXAMPLE 21: Synthesis of ¹³C-4-oxo-4H-chromene-2-carboxylic acid

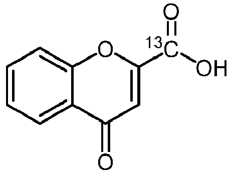

The title compound was synthesized according to general protocol from the cesium salt of 4-oxo-4H-chromene-2-carboxylic acid (32.1mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described. After the reaction and purification the enriched final compound with 72% yield (14mg) and 17% of isotopic enrichment.
**1H NMR** (400MHz, DMSO) δ 8.0 (d, *J*=9 Hz, 1H), 7.8 (t, *J*=7.7 Hz, 1H), 7.7 (d, *J*=10.3 Hz, 1H), 7.5 (t, *J*=7.7 Hz, 1H), 6.9 (s,1H)
**13C NMR** (400 MHz, DMSO) δ 177.6, 161.4, 155.3, 153.4, 135.0, 125.9, 124.9, 123.7, 118.8, 113.8
**Mass** M-1= 189.13, [M-1]+1 (13C): 17%

### EXAMPLE 22: Synthesis of ¹³C labeled aromatic compounds¹

The aromatic compounds represented below were synthesized following the general protocol for the preparation of the cesium salt and the general carbon isotopic exchange reaction described in section 3 using 3 eq of ¹³CO₂.
¹Veuillez verifier ces composes avec ceux en premiere page de votre liste et supprimer les composes qui sont en double. Attention, les rendements et les valeurs IE peuvent être differentes.

The isotopic enrichment (IE) measured by ESI mass spectrometry is indicated for each compound.

Depending on the substrate, an IE of 75% can be achieved, which is the theoretically highest incorporation possible with 3 eq of labeled *CO₂ (1 eq. of unlabeled ¹²CO₂ is released from the carboxylic acid reagent).

When 10 eq. of labeled ¹³CO₂ are used, IE can attain 90%. In addition, if ¹⁴CO₂ is used, similar enrichments are observed.

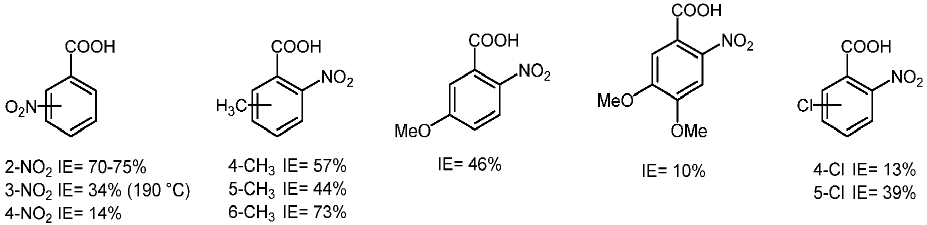

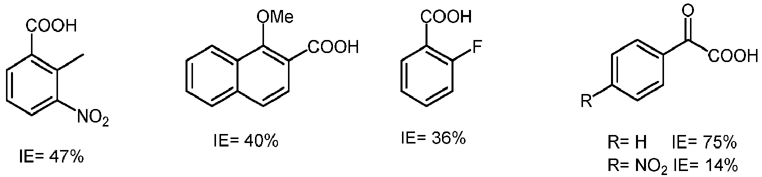

### EXAMPLE 23: Synthesis of ¹³C labeled heteroaromatic and vinylic compounds

The heteroaromatic and vinylic compounds represented below were synthesized following the general protocol for the preparation of the cesium salt and the general carbon isotopic exchange reaction described in section 3 using 3 eq of ¹³CO₂.

The isotopic enrichment (IE) measured by ESI mass spectrometry is indicated for each compound.

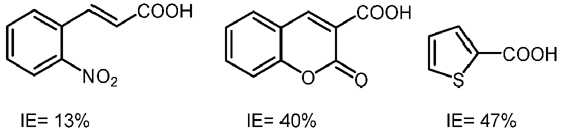

### EXAMPLE 24: Synthesis of ¹³C labeled non aromatic compounds

The non aromatic compounds represented below were synthesized following the general protocol for the preparation of the cesium salt and the general carbon isotopic exchange reaction described in section 3 using 3 eq of ¹³CO₂.

The isotopic enrichment (IE) measured by ESI mass spectrometry is indicated for each compound.

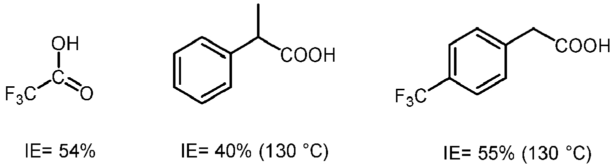

### EXAMPLE 25: Synthesis of ¹³C-5-methoxy-2-nitrobenzoic acid

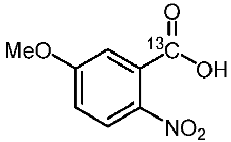

The title compound was synthesized according to general protocol from the cesium salt of 5-Methoxy-2-Nitrobenzoic acid (32.9mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described. After the reaction and purification the enriched final compound with 59% yield (11.5mg) and 46% of isotopic enrichment.
**1H NMR** (400MHz, DMSO) δ 8.0 (d, *J*=8.5 Hz, 1H), 7.2-7.1 (m, 2H), 3.9 (s, 3H),
**13C NMR** (400 MHz, DMSO) δ 166.6, 163.1, 139.5, 132 (*J*=38.1 Hz), 126.6, 115.9, 113.9, 56.5
**Mass** M-1= 196, [M-1]+1 (13C): 46%

### EXAMPLE 26: Labeling of a pharmaceutical

### A) 13C-9-fluoro-5-methyl-1-oxo-6,7-dihydro-1H,5H-pyrido[3,2,1-ij]quinoline-2-carboxylic acid (Flumequine)

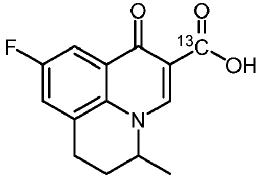

The process of the invention was applied to Flumequine, a synthetic fluoroquinolone antibiotic used to treat bacterial infections.

The title compound was also synthesized according to general protocol from the cesium salt of 9-fluoro-5-methyl-1-oxo-6,7-dihydro-1H,5H-pyrido[3,2,1-ij]quinoline-2-carboxylic acid (39.2mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture. Hence loaded with ¹³CO₂ as described. After the reaction and purification the enriched final compound with 42% yield (10.9mg) and 47% of isotopic enrichment.
**1H NMR** (400MHz, DMSO) δ 15.1 (s, 1H), 9.0 (s, 1H), 7.8 (dd, *J*=8.3, 2.9 Hz, 1H), 7.7 (dd, *J*=9, 2.9 Hz, 1H), 4.99-4.92 (m, 1H), 3.26-3.12 (m, 1H),3 (dt, *J*=17.3, 3.9 Hz, 1H), 2.2-2 (m, 2H), 1.4 (d, *J*=6.8 Hz, 3H)
**13C NMR** (400 MHz, DMSO) δ 176.9, 166, 160.2, 157.9, 147.2, 132.8, 132.3, 132.2, 121.8, 121.6, 108.0, 107.5, 107.5, 107.1, 106.7, 57.3, 25.0, 21.4, 20.0
**Mass** M-1= 260.25, [M-1]+1 (13C): 47%

### B) 13C-4-(N,N-dipropylsulfamoyl)benzoic acid (Probenecid)

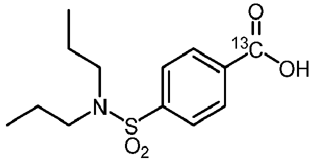

The title compound was synthetized according to general protocol from the cesium salt of 4-(N,N-dipropylsulfamoyl)benzoic acid (41.7mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described, reaction temperature 190 °C. After the reaction and purification the enriched final compound with 50% yield (14.2mg) and 25% of isotopic enrichment.
**1H NMR** (400MHz, DMSO) δ 13.4 (s, 0.8H), 8.1 (d, *J*=9.2 Hz, 2H), 7.9 (d, *J*=9.7 Hz, 2H), 3.0 (t, *J*=7.9 Hz, 4H),1.4 (dt, *J*=22.4, 7.1 Hz, 4H),0.8 (t, *J*=7.4 Hz, 6H)
**13C NMR** (400 MHz, DMSO) δ 166.1, 143.1, 134.2, 130.2, 126.9, 49.5, 21.6, 10.9 **Mass** M-1= 284.36, [M-1]+1 (13C): 25%

### C) 13C-2-(3-benzoylphenyl)propanoic acid (Ketoprofen)

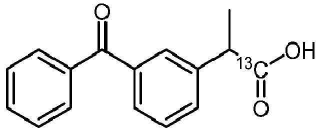

The title compound was synthesized according to general protocol from the cesium salt of 2-(3-benzoylphenyl)propanoic acid (38.5mg, 1x10⁻⁴mol), (E)-2-(4-phenyl-4,5-dihydrooxazol-2-yl)-2-(4-phenyloxazolidin-2-ylidene)acetonitrile (6.6mg, 2x10⁻⁵mol) and copper bromide (2.9mg, 2x10⁻⁵mol) after that dry DMSO 0.5ml is added to the mixture.

Hence loaded with ¹³CO₂ as described, reaction temperature 130 °C, 1h. After the reaction and purification the enriched final compound with 64% yield (16.2mg) and 10% of isotopic enrichment.
**1H NMR** (400MHz, DMSO) δ 12.4 (s, 0.8H), 7.7-7.6 (m, 4H), 7.6-7.5 (m, 5H),3.8 (dd, *J*=14.8, 7.2 Hz, 1H), 1.4 (d, *J*=6.3 Hz, 3H)
**13C NMR** (400 MHz, DMSO) δ 195.5, 175.0, 141.7, 137.0, 136.9, 132.7, 131.9, 129.6, 128.7, 128.6, 128.5, 128.3, 44.4, 18.5
**Mass** M-1= 253, [M-1]+1 (13C): 10%

## Claims

1. A process for the synthesis of a carbon labeled organic compound containing a carbon labeled carboxyl group according to formula (I) 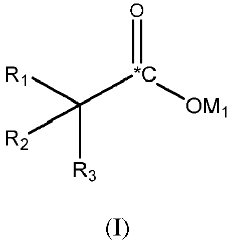 wherein
▪ *C is a ¹¹C, ¹³C or ¹⁴C isotope;
▪ R₁, R₂ and R₃ are, independently, a hydrogen atom, an aryl, a heteroaryl, a heterocycle, an alkyl, an alkyl halide, an alkene or an alkyne, said aryl, heteroaryl, heterocycle, alkene, alkyne and alkyl groups being optionally substituted, or
R₁ and R₂ form together with the carbon atom to which they are linked a carbonyl (-(C=O)- and R₃ is as defined above, or
R₁ and R₂ form together with the carbon atom to which they are linked an alkene with at least one double bond being alpha to the carboxyl group, said alkene being optionally substituted, and R₃ is as defined above, or
R₁, R₂ and R₃ form together with the carbon atom to which they are linked an aryl, a heteroaryl, or a heterocycle, said aryl, heteroaryl and heterocycle being optionally substituted;
▪ M₁ is a hydrogen atom, a silver cation (Ag⁺), an alkaline cation selected from lithium (Li⁺), sodium (Na⁺), potassium (K⁺), rubidium (Rb⁺), or cesium (Cs⁺);
**characterized in that**
- an organic compound containing a carboxyl group according to formula (II) 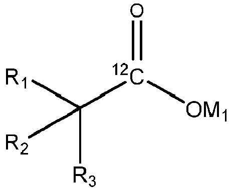 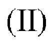 wherein R₁, R₂, R₃ and M₁ are as defined above,
- is reacted with a labeled *CO₂ wherein *C is an isotope as defined above,
- in the presence of a catalyst system comprising an inorganic salt of formula (III)
M₂(L)ₘ (III)
wherein
• M₂ is a transition metal selected from Cu, Pd, Ni, Ru, Ag, Rh, Fe, Co, Zn, Ir, Au, Pt,
• m is 1 or 2;
• L is a halogen atom selected from fluorine, chlorine, bromine, and iodide, a triflate or trifluoromethylsulfonate, a tosylate or p-toluenesulfonate, a mesylate or methanesulfonate, -CN,
and a ligand of formula (IV), formula (V) or formula (VI) 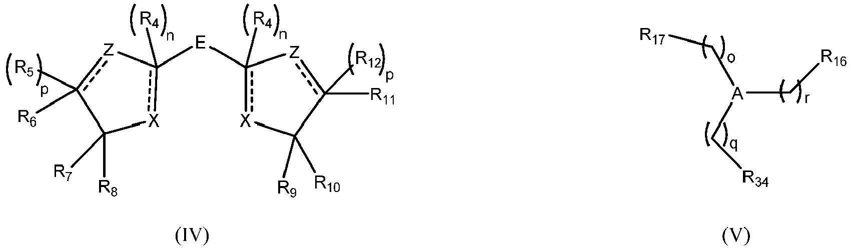 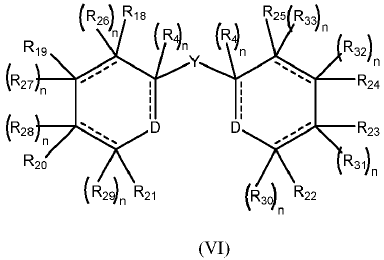 wherein
• n is 0 or 1;
• n' is 0 or 1;
• n" is 0 or 1;
• n'" is 0 or 1;
• p is 0 or 1;
• o is 0 or 1;
• q is 0 or 1;
• r is 0 or 1;
• t is 0, 1, 2 or 3;
• E is a single bond, 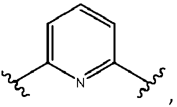 -C(R₁₃R₁₄)- with R₁₃ and R₁₄, independently being a hydrogen atom, an alkyl, an aryl, -CN, -NO₂, a halogen atom selected from F, Cl, Br, I;
• X is N(R₁₅)_{n'}, O, P, S(R₁₅)_{n'}, or P(R₁₅)_{n'} with R₁₅ being a hydrogen atom or an alkyl and with the proviso that when X is N(R₁₅)_{n'}, S(R₁₅)_{n'}, or P(R₁₅)_{n'}, and n'=n=0,is a double bond;
• Z is N(R₁₅)_{n"}, O, P, S(R₁₅)_{n"}, or P(R₁₅)_{n"}, with R₁₅ being a hydrogen atom or an alkyl and with the proviso that when Z is N(R₁₅)_{n"}, S(R₁₅)_{n"}, or P(R₁₅)_{n"}, and n"=p=0, ---- is a double bond;
• A is N or P;
• Y is a single bond, 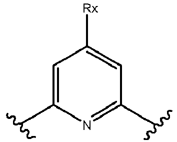 with Rₓ being a hydrogen atom or an alkyl;
• D is N(R₁₅)_{n'"}, O, P, S(R₁₅)_{n"'}, or P(R₁₅)_{n"'} with R₁₅ being a hydrogen atom or an alkyl and with the proviso that when D is N(R₁₅)_{n'"}, S(R₁₅)_{n'"}, or P(R₁₅)_{n'"} and n"'=n=0, ---- is a double bond;
• R₄, R₅, R₆, R₇, R₈, R₉, R₁₀, R₁₁ and R₁₂ are, independently, a hydrogen atom, an alkyl, an alkoxy, an aryl, or -CN, said alkyl and aryl being optionally substituted, or
R₄, R₅, R₈, R₉, and R₁₂ are, independently, a hydrogen atom, an alkyl, an alkoxy, an aryl, or -CN, R₆ and R₇ and/or R₁₀ and R₁₁ form together with the carbon atoms to which they are linked a heterocycle, said alkyl, aryl and heterocycle being optionally substituted;
• R₁₈, R₁₉, R₂₀, R₂₁, R₂₂, R₂₃, R₂₄, R₂₅, R₂₆, R₂₇, R₂₈, R₂₉, R₃₀, R₃₁, R₃₂ and R₃₃ are, independently, a hydrogen atom, an alkyl, an alkoxy, an aryl, or a -CN, said alkyl and aryl being optionally substituted, or when Y is a single bond, n=0, ---- is a double bond, R₁₈, R₂₁, R₂₂, R₂₅, R₂₆, R₂₇, R₂₈, R₂₉, R₃₀, R₃₁, R₃₂ and R₃₃ are, independently, a hydrogen atom, an alkyl, an alkoxy, an aryl, or -CN, and R₁₉ and R₂₀ and/or R₂₃ and R₂₄ form together with the carbon atoms to which they are linked an aryl, said alkyl and aryl being optionally substituted;
• R₁₆, R₁₇ and R₃₄ are, independently, a hydrogen atom, an alkyl, an alkoxy, an aryl, a heteroaryl, a heterocycle, said alkyl, aryl, heteroaryl and heterocycle being optionally substituted, -(C=S)-NR₃₅R₃₆, -(C=O)-NR₃₅R₃₆, -(CH₂)ₜ-NR₃₅R₃₆, -(CH₂)ₜ-PR₃₅R₃₆ with
R₃₅ and R₃₆ being independently, a hydrogen atom, an alkyl, an alkene, an alkyne, an aryl, a heteroaryl, a heterocycle, said alkyl, alkene, alkyne, aryl, heteroaryl and heterocycle being optionally substituted,
R₃₅ and R₃₆ form together with the nitrogen atom to which they are linked an optionally substituted heteroaryl or heterocycle.

2. The process according to claim 1, wherein the ligand is of formula (IV) 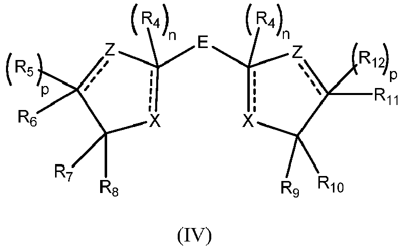 wherein
• p is 0 or 1;
• E is a single bond, 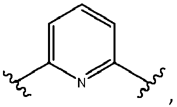 -C(R₁₃R₁₄)- with R₁₃ and R₁₄, independently being a hydrogen atom, a C₁-C₈ alkyl, or -CN;
• X is N(R₁₅)_{n'}, n' = n = 0, and ---- is a double bond;
• Z is O;
• R₅, R₆, R₇, R₈, R₉, R₁₀, R₁₁ and R₁₂ are, independently, a hydrogen atom, a C₁-C₈ alkyl, an aryl having 6 to 14 carbon atoms, or a -CN, said alkyl and aryl being optionally substituted, or
R₅, R₈, R₉, and R₁₂ are, independently, a hydrogen atom, a C₁-C₈ alkyl, an aryl having 6 to 14 carbon atoms, or -CN, R₆ and R₇ and/or R₁₀ and R₁₁ form together with the carbon atoms to which they are linked a 5 to 10 membered heterocycle, said alkyl, aryl and heterocycle being optionally substituted.

3. The process according to claim 1 or 2, wherein the ligand of formula (IV) is

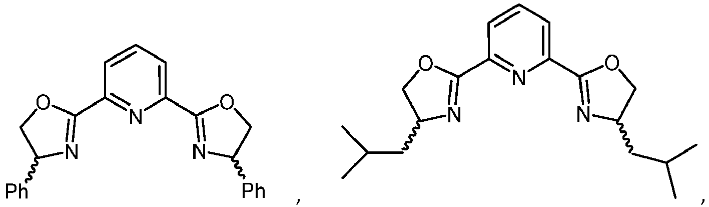

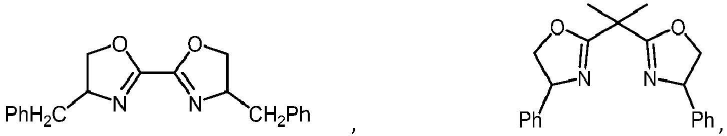

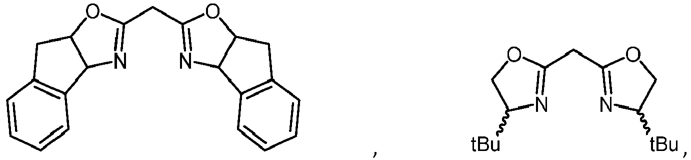

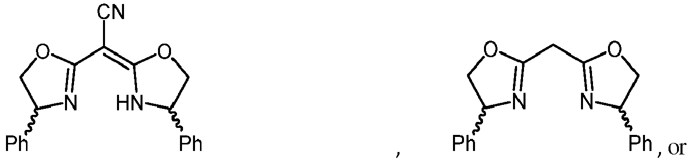

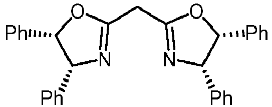

4. The process according to claim 1, wherein the ligand is of formula (V) 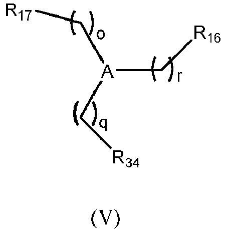 wherein
• o is 0 or 1;
• q is 0 or 1;
• r is 0 or 1;
• t is 0, 1, 2 or 3;
• A is N or P;
• R₁₆, R₁₇ and R₃₄ are, independently, a hydrogen atom, a C₁-C₈ alkyl, an aryl having 6 to 14 carbon atoms, a 5 to 10 membered heteroaryl, a 5 to 10 membered heterocycle, said alkyl, aryl, heteroaryl and heterocycle being optionally substituted, -(C=S)-NR₃₅R₃₆, -(C=O)-NR₃₅R₃₆, -(CH₂)ₜ-NR₃₅R₃₆, -(CH₂)ₜ-PR₃₅R₃₆ with
R₃₅ and R₃₆ being independently, a hydrogen atom, a C₁-C₈ alkyl, an aryl having 6 to 14 carbon atoms, a 5 to 10 membered heteroaryl, said alkyl, aryl, and heteroaryl being optionally substituted.

5. The process according to claim 1 or 4, wherein the ligand of formula (V) is 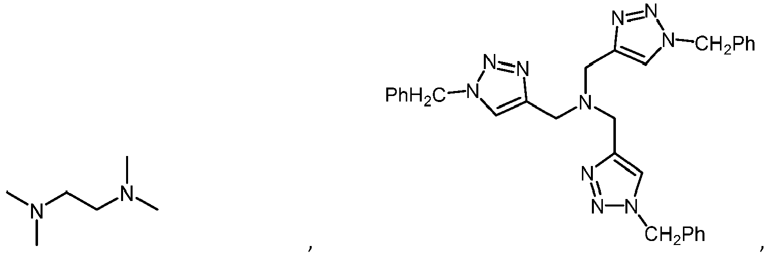 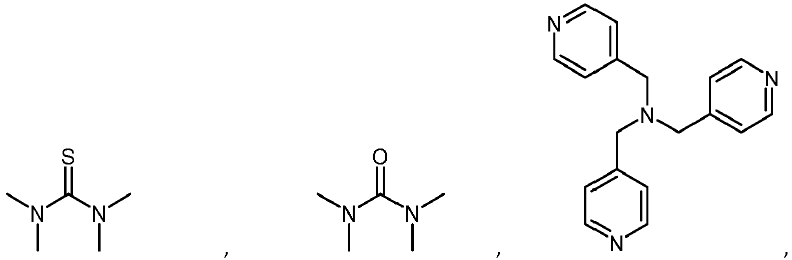 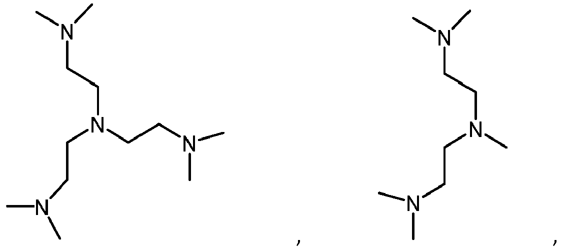 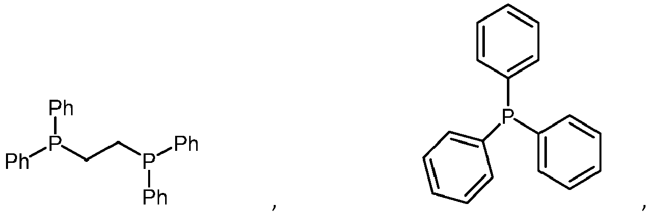 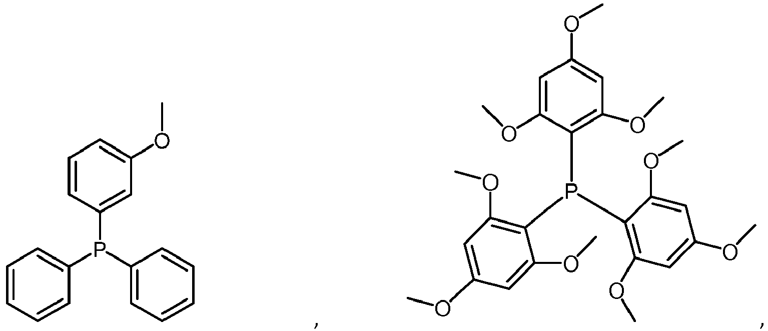 or

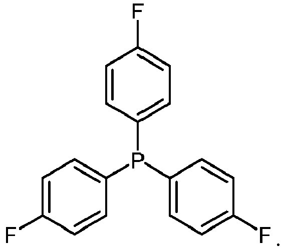

6. The process according to claim 1, wherein the ligand is of formula (VI) 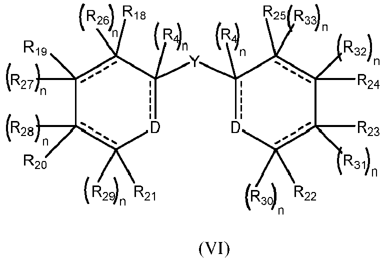 wherein
• n is 0 or 1;
• n'" is 0 or 1;
• Y is a single bond, 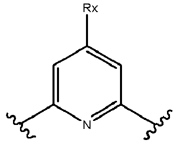 with Rₓ being a hydrogen atom or a C₁-C₈ alkyl;
• D is N(R₁₅)_{n'"}, O, P, S(R₁₅)_{n"'}, or P(R₁₅)_{n'"} with R₁₅ being a hydrogen atom or a C₁-C₈ alkyl and with the proviso that when D is N(R₁₅)_{n'"}, S(R₁₅)_{n'"}, or P(R₁₅)_{n'"} and n'"=n=0, ---- is a double bond;
• R₄ is a hydrogen atom, a C₁-C₈ alkyl, an alkoxy, an aryl having 6 to 14 carbon atoms, or -CN, said alkyl and aryl being optionally substituted;
• R₁₈, R₁₉, R₂₀, R₂₁, R₂₂, R₂₃, R₂₄, R₂₅, R₂₆, R₂₇, R₂₈, R₂₉, R₃₀, R₃₁, R₃₂ and R₃₃ are, independently, a hydrogen atom, a C₁-C₈ alkyl, a C₁-C₈ alkoxy, an aryl having 6 to 14 carbon atoms, or a -CN, said alkyl and aryl being optionally substituted, or when Y is a single bond, n=0, ---- is a double bond, R₁₈, R₂₁, R₂₂, R₂₅, R₂₆, R₂₇, R₂₈, R₂₉, R₃₀, R₃₁, R₃₂ and R₃₃ are, independently, a hydrogen atom, a C₁-C₈ alkyl, a C₁-C₈ alkoxy, an aryl having 6 to 14 carbon atoms, or -CN, and R₁₉ and R₂₀ and/or R₂₃ and R₂₄ form together with the carbon atoms to which they are linked an aryl having 6 to 14 carbon atoms, said alkyl and aryl being optionally substituted.

7. The process according to claim 1 or 6, wherein the ligand of formula (VI) is

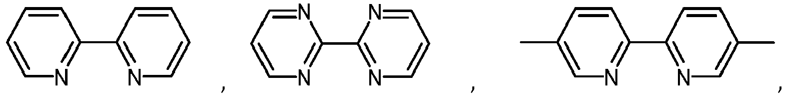

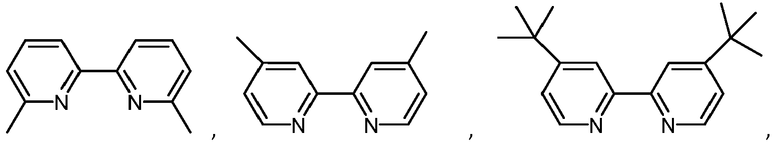

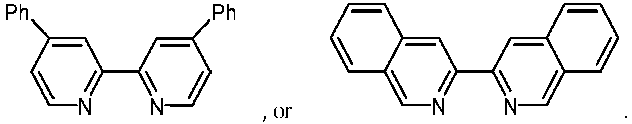

8. Process according to any one of claims 1 to 7, wherein in the inorganic salt of formula (III)
M₂(L)ₘ (III)
• M₂ is a transition metal selected from Cu, Pd, Ni, Ru, Ag, Rh, Fe, Co, Zn, Ir, Au, Pt,
• m is 1;
• L is a halogen atom selected from chlorine, bromine, and iodide, a triflate or trifluoromethylsulfonate, a tosylate or *p*-toluenesulfonate, a mesylate or methanesulfonate, -CN.

9. Process according to any one of claims 1 to 8, wherein R₁, R₂ and R₃ are, independently, a hydrogen atom, an aryl having 6 to 14 carbon atoms, a 5 to 10 membered heteroaryl, a C₁-C₈ alkyl, a C₁-C₈ alkyl halide, said aryl, heteroaryl and alkyl groups being optionally substituted.

10. Process according to any one of claims 1 to 8, wherein R₁ and R₂ form together with the carbon atom to which they are linked a carbonyl (-(C=O)- and R₃ is a hydrogen atom, an aryl having 6 to 14 carbon atoms, a 5 to 10 membered heteroaryl, a C₁-C₈ alkyl, a C₁-C₈ alkyl halide, said aryl, heteroaryl and alkyl groups being optionally substituted.

11. Process according to any one of claims 1 to 8, wherein R₁ and R₂ form together with the carbon atom to which they are linked an alkene having C₂-C₈ carbon atoms and one or more carbon-carbon double bonds with at least one double bond being alpha to the carboxyl group, said alkene being optionally substituted, and
R₃ is a hydrogen atom, an aryl having 6 to 14 carbon atoms, a 5 to 10 membered heteroaryl, a C₁-C₈ alkyl, a C₁-C₈ alkyl halide, said aryl, heteroaryl and alkyl groups being optionally substituted.

12. Process according to any one of claims 1 to 8, wherein R₁, R₂ and R₃ form together with the carbon atom to which they are linked an aryl having 6 to 14 carbon atoms, a 5 to 10 membered heteroaryl or a 5 to 10 membered heterocycle, said aryl, heteroaryl and heterocycle groups being optionally substituted.

13. Process according to any one of claims 1 to 12, wherein M₁ is a hydrogen atom, an alkaline cation selected sodium (Na⁺), potassium (K⁺), or cesium (Cs⁺).

14. Process according to any one of claims 1 to 13, wherein the organic compound containing a carboxyl group of formula (II), used for the synthesis of a carbon labeled organic compound containing a carbon labeled carboxyl group according to formula (I), is

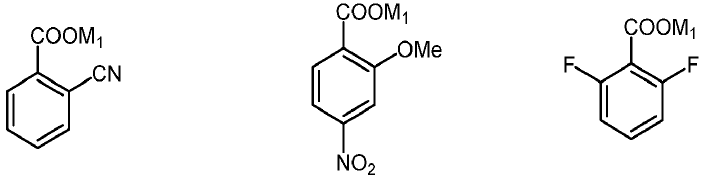

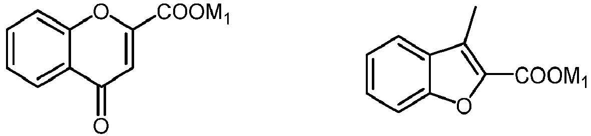

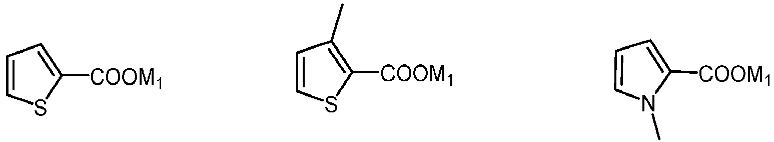

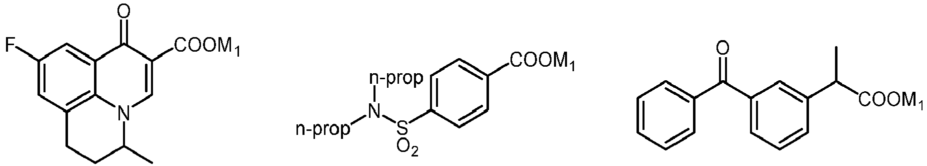

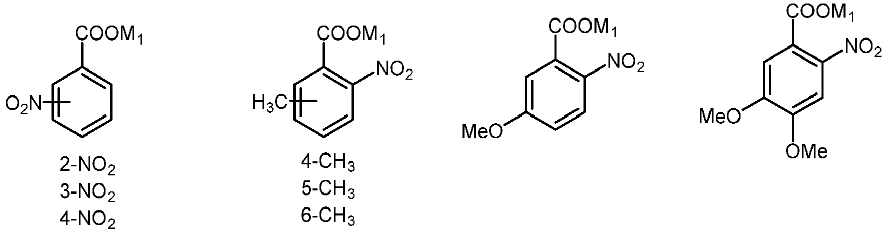

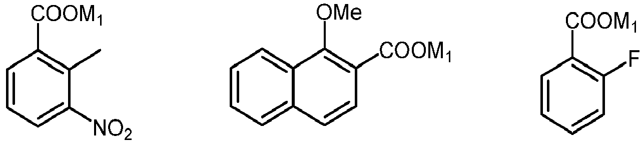

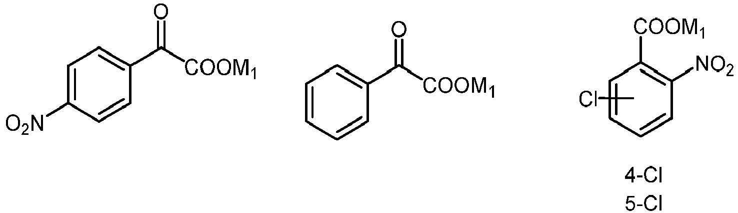

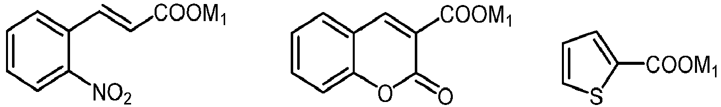

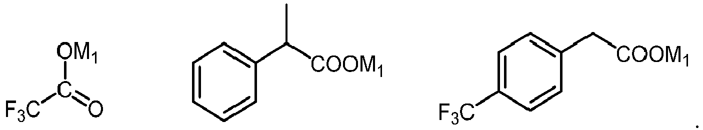

15. Process according to any one of claims 1 to 14, wherein the source of *CO₂ is a gas.

16. Process according to claim 15, wherein the *CO₂ pressure in the reaction vessel is between 0.5 to 100 bar (50 kPa to 10 MPa), in particular, between 1 and 20 bar.

17. Process according to any one of claims 1 to 16, wherein the catalyst system is present in an amount of 1 to 100 mole percent (mol %), in particular, between 5 and 25 mole percent (mol %), with respect to compound (II).

18. Process according to any one of claims 1 to 17, wherein the molar ratio of the inorganic salt of formula (III) and the ligands of formula (IV) to (V) in the catalyst system is between 1 to 100, in particular, between 1 and 20, more particularly, between 1 and 5.

19. Use of carbon labeled organic compounds containing a carbon labeled carboxyl group of formula (I) by the process according to any one of claims 1 to 18, in the manufacture of pharmaceuticals and agrochemicals.

20. A process for manufacturing labeled pharmaceuticals and agrochemicals, in particular pharmaceuticals and agrochemicals having a free carboxylic acid functionality, comprising a step of synthesis of carbon labeled organic compounds containing a carbon labeled carboxyl group of formula (I) by the process according to any one of claims 1 to 18, and optionally a step of solvent extraction and/or purification.

21. A process for producing tracers, **characterized in that** it comprises a step of synthesis of **characterized in that** it comprises a step of synthesis of carbon labeled organic compounds containing a carbon labeled carboxyl group of formula (I) by the process according according to any one of claims 1 to 18, and optionally a step of solvent extraction and/or purification.
